# FASCICULE DE BREVET EUROPEEN

(11) **EP 2 475 638 B1**
(45) Date de publication et mention de la délivrance du brevet: **27.06.2018**
(21) Numéro de dépôt: 10770550.1
(22) Date de dépôt: 10.09.2010
(51) Int. Cl.: C07C 67/03, C07C 67/31, C07C 69/708, C08G 18/36, C11C 3/00, C07C 69/732, C07D 303/40, C07D 303/44, C07D 303/42

(54) **NOUVEAU PROCÉDÉ DE PRÉPARATION DE POLYOLS ET PRODUITS TELS QU'OBTENUS**
NEUARTIGES VERFAHREN ZUR HERSTELLUNG VON POLYOLEN UND AUF DIESE WEISE GEWONNENE PRODUKTE
NOVEL METHOD FOR PREPARING POLYOLS AND PRODUCTS OBTAINED

(30) Priorité: 11.09.2009 FR 0956260
(43) Date de publication de la demande: 18.07.2012
(73) Titulaire: Centre National de la Recherche Scientifique (C.N.R.S.), 75016 Paris (FR)
(72) Inventeur: CRAMAIL, Henri, F-33350 Sainte Terre (FR); BOYER, Aurélie, F-33000 Bordeaux (FR); CLOUTET, Eric, F-33880 Saint Caprais De Bordeaux (FR); BAKHIYI, Rachida, F-33700 Merignac (FR); ALFOS, Carine, F-33600 Pessac (FR)
(74) Mandataire: Lavoix
(86) Numéro de dépôt international: PCT/FR2010/051894
(87) Numéro de publication internationale: WO 2011/030076

(56) Documents cités:
- WO-A1-03/093215
- US-A- 5 324 846
- US-A- 5 380 886
- US-A- 6 107 433
- US-A1- 2007 232 816

## Description

La présente invention concerne un nouveau procédé de polyols, notamment de diols, ainsi que les nouveaux polyols tels qu'obtenus.

Il existe différentes approches pour la synthèse de polymères à partir d'huiles végétales. La première, la plus répandue, consiste à considérer les triglycérides comme matériaux de base, ceux-ci pouvant être époxydés puis par exemple alcoolisés ou hydroformylés, afin de les rendre fonctionnels et polymérisables.

Une huile est un mélange de triglycérides (triesters) formés par condensation des acides gras et du glycérol. Le nombre élevé de types d'acides gras (jusqu'à 24) présents dans chaque corps gras et les multiples possibilités de leurs combinaisons avec les molécules de glycérol font que les corps gras sont des mélanges très complexes de composés dont les propriétés varient d'une huile à une autre. La nature des triglycérides peut donc varier au sein d'une même huile.

Les sites réactifs présents dans un triglycéride sont principalement les doubles liaisons et les fonctions ester. La réactivité des doubles liaisons permet d'introduire des fonctions hydroxyles, permettant ainsi l'accès à des monomères plurihydroxylés. Il est néanmoins impossible d'obtenir des triglycérides ayant des structures et des fonctionnalités parfaitement définies.

La synthèse de polyols issus d'huile végétale est bien décrite dans la littérature car ces derniers constituent d'excellents précurseurs pour la synthèse de polymères. Ces matériaux gagnent en popularité en raison de l'origine naturelle des précurseurs et des propriétés attractives apportées par la structure et la composition des huiles végétales. Les sites réactifs dans tous les corps gras sont les fonctions esters et les doubles liaisons. Certaines huiles possèdent également d'autres groupements comme des hydroxyles ou des époxydes.

Les doubles liaisons de ces composés ne sont généralement pas assez réactives pour servir de sites de polymérisation radicalaire. Néanmoins, à haute température (330°C), les doubles liaisons peuvent migrer le long du squelette pour former des sites conjugués, ce qui facilite les condensations de type Diels-Alder. Des oligomères ont été synthétisés par vulcanisation d'huiles avec du monochlorure de soufre et utilisés comme additifs dans l'industrie des gommes par exemple. Des oligomères ont également été synthétisés par polymérisation cationique en présence de trifluorure de bore (Croston et al., J. Amer. Oil Chem. Soc. 1952, 331-333), pour des applications dans des formulations d'encres. D'autres réactions faisant intervenir les doubles liaisons, comme la polymérisation par métathèse, ont permis d'obtenir des oligomères (Refvik et al., J.Amer. Oil Chem. Soc. 1999, 76, 93-98) et les matériaux qui en sont issus sont rarement exploitables car très mal définis.

Il est donc nécessaire de mieux contrôler la fonctionnalisation des huiles végétales.

Comme déjà indiqué, la présence de doubles liaisons sur le squelette permet l'introduction de groupements hydroxyles. Celle-ci peut être réalisée par oxydation directe des doubles liaisons, qui consiste à faire passer un courant d'oxygène à travers l'huile chauffée à 135°C (G., Soucek et al. "Spectroscopic investigation of blowing process of soybean oil", Surface Coatings International, Part B, Coatings Transactions. 2003, 86: 221-229). Le contrôle de l'oxydation n'est pas satisfaisant et de nombreux sous-produits sont formés tels que des peroxydes, aldéhydes, cétones, des scissions de chaînes, etc. Le seul avantage de ces polyols est leur faible coût de revient et leur synthèse réalisée en une seule étape, malgré les nombreux traitements appliqués au produit final (odeurs, indice d'acide élevé, couleur foncée, etc.).

Un catalyseur organométallique peut également être utilisé afin de mieux contrôler la réaction d'oxydation (WO2006/094227 ; WO2007/143135) en présence d'un oxydant.

Des polyols possédant des hydroxyles primaires peuvent être préparés par hydroformylation des insaturations (Guo et al., J. of Polymers and the Environment. 2002, 10: 49-52). Ce procédé fait intervenir une réaction entre du monoxyde de carbone et du dihydrogène, entraînant la formation d'un groupement aldéhyde qui est converti en hydroxyle par hydrogénation. Les catalyseurs à base de rhodium généralement utilisés sont très efficaces (conversions proches de 100%) mais aussi très coûteux. A l'inverse, les catalyseurs à base de cobalt sont bon marché mais moins efficaces. L'ozonolyse des doubles liaisons permet également d'obtenir des polyols ayant des groupements hydroxyles terminaux (Guo et al., J. of Polymer Sci., 2000, 38: 3900-3910). L'ozone passe à travers une solution d'huile végétale et d'éthylène glycol, en présence d'un catalyseur alcalin.

Une autre voie d'accès aux polyols consiste à réaliser une réaction préalable d'époxydation des insaturations. De nombreux travaux décrits dans la littérature décrivent l'époxydation de corps gras (Swern, et al., J. Am. Chem. Soc. 1944, 66, 1925-1927; Findley et al., J. Am. Chem. Soc. 1945, 67, 412-414; US 5 026 881; US 3 328 430; Petrovic' et al., Eur. J. Lipid Sci. Technol. 2002, 104: 293-299 et US 4 647 678). Petrovic a récemment démontré la possibilité de réaliser l'époxydation d'huile végétale par voie enzymatique (̌̌VIč̌̌̌̌̌̌̌̌̌̌̌̌ ̌ek, Ť̌. et al., J. Amer. Oil Chem. Soc. 2006, 83: 247-252) ou catalysée par une résine échangeuse d'ions (Sinadinovic'-Fis ̌er et al., J. Amer. Oil Chem. Soc. 2001, 78: 725). Néanmoins, la voie la plus courante est l'utilisation d'un peracide formé in situ, généralement du peroxyde d'hydrogène en présence d'un acide carboxylique (le plus souvent de l'acide formique en quantité catalytique). La réaction est conduite entre 50 et 80°C pendant 1 à 4 heures.

Il est important de souligner que l'époxydation de corps gras possédant déjà un alcool primaire en bout de chaîne n'a jamais été réalisée à partir de peracide formé in situ. Cette réaction ne peut pas se faire dans les mêmes conditions que précédemment en raison de réactions secondaires entre l'acide carboxylique et l'alcool terminal.

Les huiles végétales époxydées ont été utilisées comme intermédiaires dans de nombreuses synthèses. Le groupe de Petrovic décrit l'ouverture des époxydes par des alcools, des acides inorganiques et par hydrogénation sous catalyse acide (Guo et al., J. Polym. Sci. Part A: Polym. Chem. 2000, 38: 3900-3910). Des triglycérides époxydés ont été modifiés par réaction avec HCI ou HBr en présence d'acétone (solvant), par hydrogénation avec H₂ en présence d'isopropanol et de nickel de Raney comme catalyseur, et enfin par du méthanol en présence d'isopropanol et d'un catalyseur acide (ex acide fluoborique).

Ces triglycérides fonctionnels possèdent des réactivités différentes ; celui issu de l'ouverture par le méthanol étant le plus réactif vis-à-vis des isocyanates pour la chimie des polyuréthanes. Petrovic augmente la réactivité des polyols obtenus par éthoxylation (ouverture de l'oxyde d'éthylène par l'alcool secondaire sous catalyse acide), ce qui convertit les alcools secondaires en alcools primaires. Il note cependant que l'utilisation d'un catalyseur acide entraîne des réactions secondaires, comme la formation d'ester méthylique lors de l'ouverture de l'époxyde avec du méthanol. La solution réside dans l'utilisation d'un catalyseur spécifique à l'ouverture de l'époxyde et opérationnel à des températures plus basses afin d'éviter des réactions secondaires de couplages. Enfin, il est intéressant de noter que la demande de brevet américain, publiée sous le numéro 2006/7045577, décrit la synthèse de polyuréthane issu d'huile de soja selon un processus en deux étapes : (i) l'huile est époxydée à partir des méthodes conventionnelles utilisant un peracide et (ii) l'huile de soja époxydée réagit avec du dioxyde de carbone pour donner une huile végétale carbonatée. La réaction de ce produit avec une diamine permet l'accès à un polyuréthane sans utiliser d'isocyanates.

Le document US 2007/232816 décrit la préparation de polyols à partir de monoglycéride d'acides gras insaturés, comprenant une étape d'époxydation en présence d'un peracide et une étape d'ouverture du cycle époxyde avec un polyol. L'étape d'époxydation est effectuée via la réaction d'un peracide avec un monoglycéride d'acide gras insaturé.

La présente invention a pour but de fournir un procédé de préparation de polyols à partir d'esters d'huile végétale permettant de s'affranchir des inconvénients susmentionnés.

La présente invention a également pour but de fournir un procédé de préparation de polyols comprenant l'utilisation de catalyseurs répondant mieux aux attentes environnementales que la plupart des catalyseurs homogènes utilisés et qui limitent les réactions secondaires.

La présente invention a également pour but de fournir un procédé qui, contrairement aux procédés de l'art antérieur qui concernent la transformation chimique à partir de triglycérides ayant des structures mal définies, consiste en une voie simple et efficace de modification chimique de monoesters ou de triglycérides pour l'obtention de précurseurs fonctionnels à fonctionnalité contrôlée.

La présente invention a pour but de fournir un procédé de préparation simple en trois étapes via des mono- ou des diesters d'huile végétale.
Un but de la présente invention est de fournir un procédé en trois étapes permettant l'accès à de nouveaux synthons, mono-esters ou di-esters, tous au moins bifonctionnels (polyols) et possédant des structures bien définies et faisant appel à une catalyse propre.

Il est décrit un procédé de préparation d'un polyol répondant à la formule générale (I") suivante : dans laquelle : 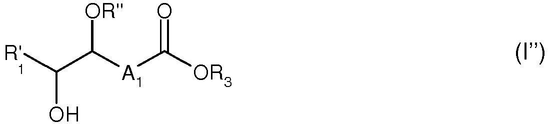
- R'₁ représente H ou un groupe alkyle, linéaire ou ramifié, comprenant de 2 à 14 atomes de carbone, ledit groupe alkyle pouvant éventuellement être substitué par un ou plusieurs groupes ORₐ, Rₐ représentant H ou un groupe R' tel que défini ci-dessous,
- A₁ représente un radical alkylène divalent linéaire ou ramifié, comprenant de 2 à 14 atomes de carbone,
- R" représente :
   - un groupe alkyle R', linéaire ou ramifié, comprenant de 1 à 18 atomes de carbone, ou
   - un groupe de formule -A₂-OH, A₂ représentant un radical alkylène divalent linéaire ou ramifié, comprenant de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
   A₂ représentant de préférence un radical de formule -CH₂-A₃-CH₂-, A₃ représentant un groupe de formule -(CH₂OCH₂)ₙ-, n représentant un nombre entier compris de 1 à 100, et de préférence égal à 6, 13 ou 45, ou un radical phénylène,
- R₃ représente :
   - un groupe alkyle R₂, linéaire ou ramifié, comprenant de 1 à 10, de préférence de 1 à 6, atomes de carbone, ou
   - un groupe de formule -A₂-O-Y', A₂ étant tel que défini ci-dessus et Y' représentant un atome d'hydrogène ou un groupe de formule (A') 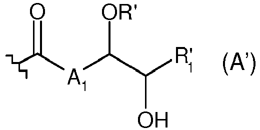
      A₁, R' et R'₁ étant tels que définis ci-dessus dans la formule (I"),
      étant entendu que lorsque R" est un groupe R' alors R₃ représente un groupe de formule -A₂-O-Y', et que lorsque R" est un groupe -A₂-OH alors R₃ représente un groupe R₂,
      ledit procédé comprenant les étapes suivantes :
         a) une étape d'époxydation du composé de formule (IV") suivante : 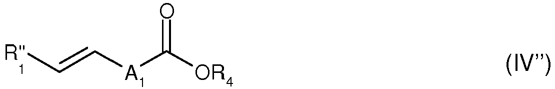
            A₁ étant tel que défini ci-dessus dans la formule (I"),
            R"₁ représentant H ou un groupe alkyle, linéaire ou ramifié, comprenant de 2 à 14 atomes de carbone, ledit groupe alkyle pouvant éventuellement contenir une ou deux doubles liaisons, et ledit groupe alkyle pouvant également le cas échéant être substitué par un groupe OH,
            R₄ représentant un groupe alkyle R₂ tel que défini ci-dessus, ou un groupe de formule -A₂-O-Y₁', A₂ étant tel que défini ci-dessus et Y'₁ représentant un atome d'hydrogène ou un groupe de formule (A'₁) 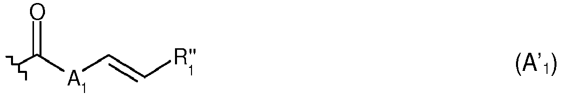
            A₁ étant tel que défini ci-dessus dans la formule (I") et R"₁ étant tel que défini ci-dessus dans la formule (IV"),
            pour obtenir un composé de formule (V") suivante : 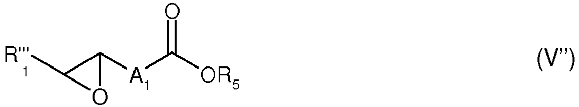
            A₁ étant tel que défini ci-dessus dans la formule (I"),
            R'"₁ représentant H ou un groupe alkyle, linéaire ou ramifié, comprenant de 2 à 14 atomes de carbone, ledit groupe alkyle pouvant éventuellement contenir un ou deux groupes époxydes, et ledit groupe alkyle pouvant également le cas échéant être substitué par un groupe OH,
            R₅ représentant un groupe alkyle R₂ tel que défini ci-dessus, ou un groupe de formule -A₂-O-Y₂', A₂ étant tel que défini ci-dessus et Y'₂ représentant un atome d'hydrogène ou un groupe de formule (A'₂) 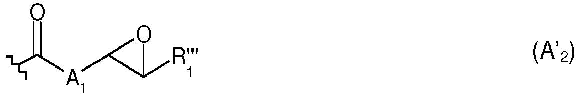
            A₁ étant tel que défini ci-dessus dans la formule (I") et R'"₁ étant tel que défini ci-dessus dans la formule (V"), et
         b) une étape d'ouverture du cycle époxyde du composé de formule (V") avec un alcool de formule R"OH, R" étant tel que défini ci-dessus, pour obtenir un composé de formule (I") telle que définie ci-dessus, et
         c) une étape de récupération du composé de formule (I") telle que définie ci-dessus.

Sont décrits des composés de départ de formule (IV") englobant à la fois les composés (IV') et (IV"-1) :

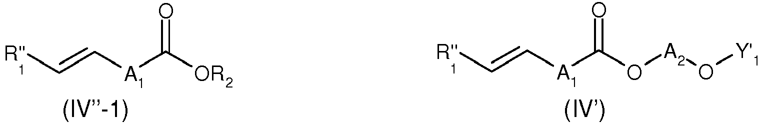

Le composé (IV"-1) décrit est obtenu par transestérification d'une huile végétale, notamment huile de tournesol, de colza ou de ricin, avec un alcool R₂OH, et le composé (IV') est obtenu par transestérification du composé (IV"-1) avec un diol HO-A₂-OH.

Lorsque l'étape d'époxydation a) décrite est effectuée sur un composé (IV"-1), on obtient alors un composé (V"-1) :

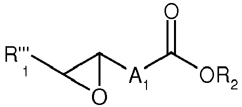

Lorsque l'étape d'époxydation a) décrite est effectuée sur un composé (IV'), on obtient alors un composé (V') :

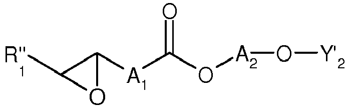

Lorsque l'étape d'ouverture du cycle b) décrite est effectuée sur un composé (V"-1), on obtient alors un composé (I"-1) :

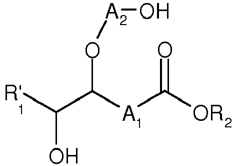

Le composé de formule (I"-1) décrit est un composé de type monoester comprenant au moins deux fonctions hydroxyles. Il peut éventuellement contenir d'autres fonctions hydroxyles en fonction de la nature de R'₁.

Lorsque l'étape d'ouverture du cycle b) décrite est effectuée sur un composé (V'), on obtien alors un composé (I') :

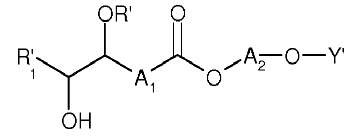

Le composé de formule (I') décrit est un composé de type monoester ou diester (en fonction de la nature de Y') comprenant au moins deux fonctions hydroxyles. Il peut éventuellement contenir d'autres fonctions hydroxyles en fonction de la nature de R'₁.

Le produit de départ (IV") décrit contient un groupe R"₁ qui peut éventuellement contenir une ou deux doubles liaisons. Ainsi, lors de l'étape d'époxydation conduisant au composé (V"), ces doubles liaisons peuvent être également époxydées d'où la nature du groupe R"'₁. Enfin, lors de l'étape d'ouverture du cycle, les groupes époxydes présents dans le groupe R'"₁ sont alors également modifiés, d'où la définition susmentionnée de R'₁ et donc la présence éventuelle d'un ou deux groupes hydroxyles dans R'₁.

Il est également décrit un procédé de préparation d'un polyol répondant à la formule générale suivante (I"-1) : dans laquelle : 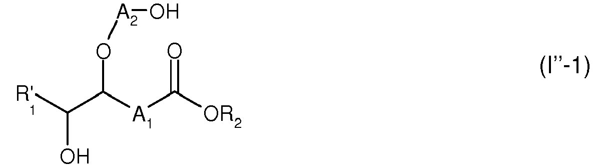
- R'₁ représente H ou un groupe alkyle, linéaire ou ramifié, comprenant de 2 à 14 atomes de carbone, ledit groupe alkyle pouvant éventuellement être substitué par un ou plusieurs groupes ORₐ, Rₐ représentant H ou un groupe R' tel que défini ci-dessous,
- A₁ représente un radical alkylène divalent linéaire ou ramifié, comprenant de 2 à 14 atomes de carbone,
- A₂ représente un radical alkylène divalent linéaire ou ramifié, comprenant de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
   A₂ représentant de préférence un radical de formule -CH₂-A₃-CH₂-, A₃ représentant un groupe de formule -(CH₂OCH₂)ₙ-, n représentant un nombre entier compris de 1 à 100, et de préférence égal à 6, 13 ou 45, ou un radical phénylène,
- R₂ représente un groupe alkyle, linéaire ou ramifié, comprenant de 1 à 10, de préférence de 1 à 6, atomes de carbone,
   ledit procédé comprenant les étapes suivantes :
   a) une étape d'époxydation d'un composé de formule (II'-1) suivante : 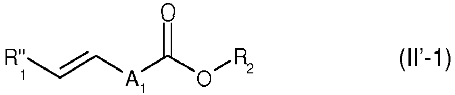
      R"₁ représentant H ou un groupe alkyle, linéaire ou ramifié, comprenant de 2 à 14 atomes de carbone, ledit groupe alkyle pouvant éventuellement contenir une ou deux doubles liaisons, et ledit groupe alkyle pouvant également le cas échéant être substitué par un groupe OH,
      A₁ et R₂ étant tels que définis ci-dessus dans la formule (I"-1),
      pour obtenir un composé de formule (IV"-1) suivante : 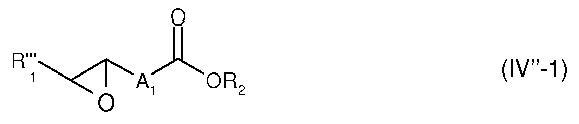
      A₁ et R₂ étant tels que définis ci-dessus dans la formule (I"-1),
      R'"₁ représentant H ou un groupe alkyle, linéaire ou ramifié, comprenant de 2 à 14 atomes de carbone, ledit groupe alkyle pouvant éventuellement contenir un ou deux groupes époxydes, et ledit groupe alkyle pouvant également le cas échéant être substitué par un groupe OH,
   b) une étape d'ouverture du cycle époxyde avec un diol de formule HO-A₂-OH, A₂ étant tel que défini ci-dessus, pour obtenir un composé de formule (I"-1) telle que définie ci-dessus, et
   c) une étape de récupération du composé de formule (I"-1) telle que définie ci-dessus. Un procédé de préparation d'un polyol répondant à la formule générale (I') suivante est également décrit : 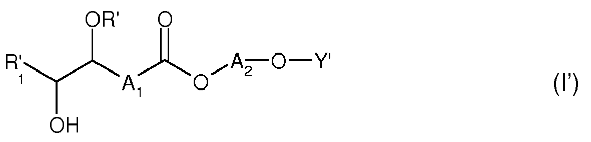 dans laquelle :
      R'₁ représente H ou un groupe alkyle, linéaire ou ramifié, comprenant de 2 à 14 atomes de carbone, ledit groupe alkyle pouvant éventuellement être substitué par un ou plusieurs groupes ORₐ, Rₐ représentant H ou un groupe R' tel que défini ci-dessous,
      R' représente un groupe alkyle, linéaire ou ramifié, comprenant de 1 à 18 atomes de carbone,
      A₁ représente un radical alkylène divalent linéaire ou ramifié, comprenant de 2 à 14 atomes de carbone,
      A₂ représente un radical alkylène divalent linéaire ou ramifié, comprenant de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
      A₂ représentant de préférence un radical de formule -CH₂-A₃-CH₂-, A₃ représentant un groupe de formule -(CH₂OCH₂)ₙ-, n représentant un nombre entier compris de 1 à 100, et de préférence égal à 6, 13 ou 45, ou un radical phénylène,
      Y' représente un atome d'hydrogène ou un groupe de formule (A') 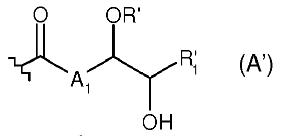
      A₁, R' et R'₁ étant tels que définis ci-dessus dans la formule (I'),
      ledit procédé comprenant les étapes suivantes :
         a) une étape de transestérification d'un composé de formule (II') suivante : 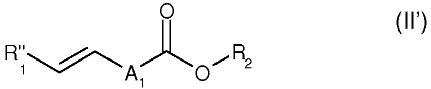
            R"₁ représentant H ou un groupe alkyle, linéaire ou ramifié, comprenant de 2 à 14 atomes de carbone, ledit groupe alkyle pouvant éventuellement contenir une ou deux doubles liaisons, et ledit groupe alkyle pouvant également le cas échéant être substitué par un groupe OH,
            A₁ étant tel que défini ci-dessus dans la formule (I'),
            R₂ représentant un groupe alkyle, linéaire ou ramifié, comprenant de 1 à 10, de préférence de 1 à 6, atomes de carbone,
            avec un diol de formule (III) suivante :

               HO-A₂-OH (III)
            pour obtenir un composé de formule (IV') suivante : 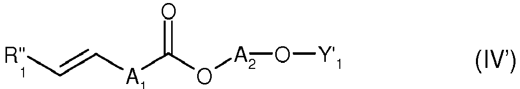
            A₁, A₂ et R"₁ étant tels que définis ci-dessus,
            Y'₁ représentant un atome d'hydrogène ou un groupe de formule (A'₁) 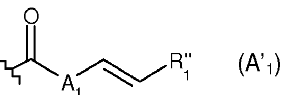
            A₁ étant tel que défini ci-dessus dans la formule (I') et R"₁ étant tel que défini ci-dessus dans la formule (II'),
         b) une étape d'époxydation du composé de formule (IV') susmentionnée pour obtenir un composé de formule (V') suivante : 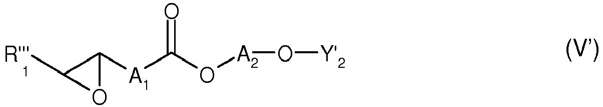
            A₁ et A₂ étant tels que définis ci-dessus dans la formule (I'),
            R'"₁ représentant H ou un groupe alkyle, linéaire ou ramifié, comprenant de 2 à 14 atomes de carbone, ledit groupe alkyle pouvant éventuellement contenir un ou deux groupes époxydes, et ledit groupe alkyle pouvant également le cas échéant être substitué par un groupe OH,
            Y'₂ représentant un atome d'hydrogène ou un groupe de formule (A'₂) 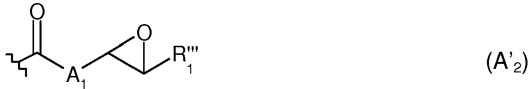
            A₁ étant tel que défini ci-dessus dans la formule (I') et R'"₁ étant tel que défini ci-dessus dans la formule (V'),
         c) une étape d'ouverture du cycle époxyde avec un alcool de formule R'OH, R' étant tel que défini ci-dessus, pour obtenir un composé de formule (I') telle que définie ci-dessus, et
         d) une étape de récupération du composé de formule (I') telle que définie ci-dessus.

Ainsi, le procédé de l'invention consiste à synthétiser de nouveaux polymères à partir de mono-esters d'acides gras. Ces derniers sont généralement obtenus par exemple par transestérification des triglycérides avec un alcool court (R₂OH, R₂ étant tel que défini ci-dessus), de préférence avec du méthanol ou de l'éthanol.

Ces esters, et plus particulièrement les esters méthyliques ou éthyliques de tournesol ou de ricin, ont donc été utilisés comme 'synthons' de base dans la présente invention.

En utilisant une variété d'huile de tournesol dont la teneur en acide oléique est particulièrement élevée et en séparant par distillations fractionnées les différents esters méthyliques de l'huile de tournesol, on obtient des esters méthyliques d'acide oléique (une seule double liaison par chaîne grasse) de pureté élevée. C'est à partir de ce précurseur monofonctionnel qu'il est ensuite possible d'apporter un nombre choisi de groupes hydroxyles et ainsi de contrôler la fonctionnalité de ce 'synthon' monomère. Une structure bien définie de tels monomères est en effet indispensable à l'élaboration de matériaux polymères présentant des propriétés contrôlées et reproductibles. Les polymères désirés étant linéaires, on a par exemple cherché à créer des monomères au moins difonctionnels (di-OH) à partir d'esters méthyliques de tournesol oléique.

Dans le cadre de la présente invention, les inventeurs se sont focalisés sur l'utilisation de catalyseurs répondant mieux aux attentes environnementales que la plupart des catalyseurs homogènes utilisés et qui limitent les réactions secondaires. En ce sens, la catalyse hétérogène, notamment l'utilisation de résines échangeuses d'ions, est particulièrement intéressante. De telles résines sont des catalyseurs efficaces pour favoriser de nombreuses réactions comme des estérifications, éthérifications, transalkylations et alkylations (G.D. Yadav, P.H. Mehta, Indust. Eng. Chem. Res. 1994, 33: 2198-2208). La catalyse hétérogène offre aussi des avantages non négligeables par rapport à la catalyse homogène (recyclage, sélectivité et non toxicité). Ce nouveau procédé a donc été appliqué à l'ouverture de corps gras époxydés.

Il est décrit plusieurs voies pour l'obtention de polyols :
- une voie en trois étapes *via* des monoesters comprenant en particulier la transestérification d'esters méthyliques d'huile végétale pour former des monoesters, époxydation puis ouverture des cycles ; et
- une voie en trois étapes *via* des diesters comprenant en particulier la transestérification d'esters méthyliques d'huile végétale pour former des diesters, époxydation et ouverture de cycles.

Les différentes réactions mises en jeu pour les deux premiers procédés sont (i) la transestérification du groupement ester par des diols permettant de greffer une première fonction hydroxyle primaire (étape a) susmentionnée), (ii) l'époxydation de la double liaison (étape b) susmentionnée), suivie de (iii) l'ouverture de l'époxyde apportant la deuxième fonction hydroxyle (étape c) susmentionnée).

Ainsi, la présente invention permet de préparer des polyols répondant à l'une des deux formules suivantes : 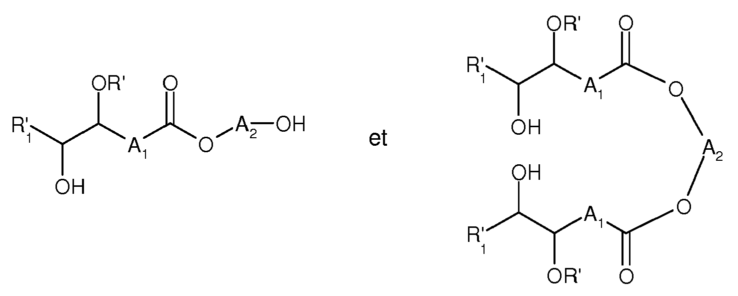 à savoir, d'une part, des diols non symétriques avec une fonction OH primaire (en bout de chaîne) et une fonction OH secondaire, et, d'autre part, des diols symétriques avec deux fonctions OH secondaires.

Plus particulièrement, le procédé susmentionné concerne la préparation de diols. Ainsi, la présente invention concerne un procédé de préparation d'un polyol répondant à la formule générale (I) suivante : 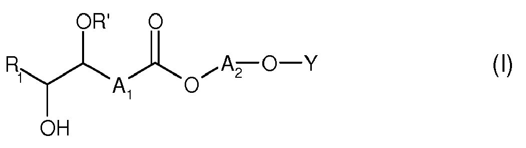 dans laquelle :
- R₁ représente H ou un groupe alkyle, linéaire ou ramifié, comprenant de 2 à 14 atomes de carbone,
- R' représente un groupe alkyle, linéaire ou ramifié, comprenant de 1 à 18 atomes de carbone,
- A₁ représente un radical alkylène divalent linéaire ou ramifié, comprenant de 2 à 14 atomes de carbone,
- A₂ représente un radical alkylène divalent linéaire ou ramifié, comprenant de 1 à 10 atomes de carbone, le cas échéant comprenant un ou plusieurs substituants choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
- Y représente un atome d'hydrogène ou un groupe de formule (A) 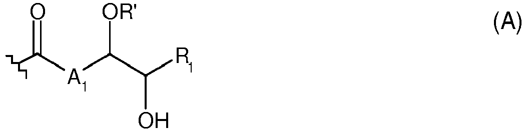
   A₁, R' et R₁ étant tels que définis ci-dessus,
   ledit procédé comprenant les étapes suivantes :
      a) une étape de transestérification d'un composé de formule (II) suivante : 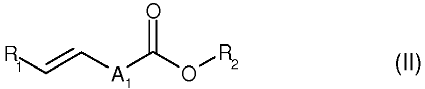
         R₁ et A₁ étant tels que définis ci-dessus, et R₂ représente un groupe alkyle, linéaire ou ramifié, comprenant de 1 à 10, de référence de 1 à 6, atomes de carbone,
         avec un diol de formule (III) suivante :

            HO-A₂-OH (III)
         pour obtenir un composé de formule (IV) suivante : 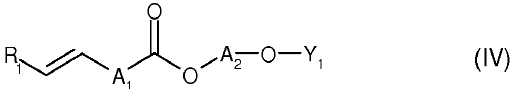
         A₁, A₂ et R₁ étant tels que définis ci-dessus dans la formule (I),
         Y₁ représentant un atome d'hydrogène ou un groupe de formule (A₁) 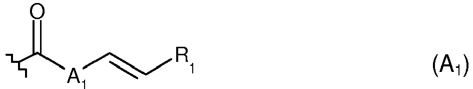
         A₁ et R₁ étant tel que défini ci-dessus,
      b) une étape d'époxydation du composé de formule (IV) susmentionnée pour obtenir un composé de formule (V) suivante : 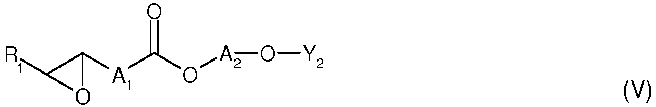
         A₁, A₂ et R₁ étant tels que définis ci-dessus,
         Y₂ représentant un atome d'hydrogène ou un groupe de formule (A₂) 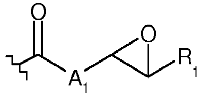 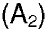
         A₁ et R₁ étant tels que définis ci-dessus,
      c) une étape d'ouverture du cycle époxyde avec un alcool de formule R'OH, R' étant tel que défini ci-dessus, pour obtenir un composé de formule (I) telle que définie ci-dessus, et
      d) une étape de récupération du composé de formule (I) telle que définie ci-dessus.

Il est décrit un procédé tel que défini ci-dessus, pour la préparation d'un diol répondant à la formule générale (I"-2) suivante : dans laquelle : 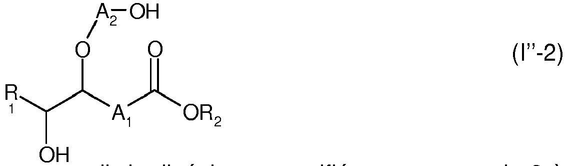
R₁ représente H ou un groupe alkyle, linéaire ou ramifié, comprenant de 2 à 14 atomes de carbone,
R₂, A₁ et A₂ sont tels que définis ci-dessus dans la formule (I"-1),
ledit procédé comprenant les étapes suivantes :
   a) une étape d'époxydation du composé de formule (II'-2) : 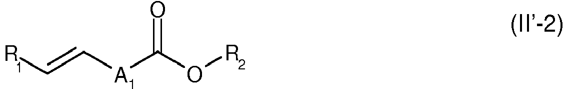
      A₁, R₁ et R₂ étant tels que définis ci-dessus dans la formule (I"-2),
      pour obtenir un composé de formule (IV"-2) suivante : 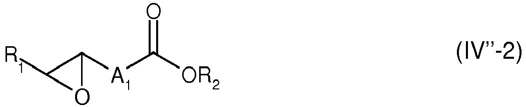
      A₁, R₁ et R₂ étant tels que définis ci-dessus,
   b) une étape d'ouverture du cycle époxyde du composé de formule (IV"-2) telle que définie ci-dessus avec un diol de formule HO-A₂-OH, A₂ étant tel que défini ci-dessus, pour obtenir un composé de formule (I"-2) telle que définie ci-dessus, et
   c) une étape de récupération du composé de formule (I"-2) telle que définie ci-dessus.

Comme indiqué précédemment, les diols de l'invention de formule (I) présentent la particularité d'être issus de corps gras naturels et de présenter une fonctionnalité exacte de deux.

Le procédé de l'invention de préparation des composés (I) comprend une première étape de transestérification effectuée en catalyse hétérogène (oxyde de magnésium ou autre catalyseur hétérogène) et de préférence en l'absence de solvant (synthèse propre).

La deuxième étape du procédé de l'invention de préparation des composés (I) est une époxydation et cette synthèse est particulière en raison de la présence du groupement hydroxyle terminal des monoesters. L'époxydation nécessite l'utilisation d'un peracide déjà formé afin d'éviter une réaction secondaire avec l'alcool en bout de chaîne et l'ouverture de l'époxyde n'est possible qu'en conditions relativement douces pour inhiber la formation de couplages. La spécificité de cette deuxième étape consiste en l'utilisation de peracide préformé. Enfin, la troisième étape du procédé de l'invention de préparation des composés (I) consiste à ouvrir l'époxyde par des alcools (méthanol, éthanol, propanol, etc) sous catalyse acide. La spécificité de cette étape consiste en ce qu'elle met en oeuvre de préférence une résine échangeuse d'ions recyclable et sélective et est effectuée préférentiellement en l'absence de solvant. Il est également important de noter que ces trois réactions se succèdent sans purification intermédiaire (une seule purification peut être effectuée à la fin, ce qui facilite la mise en place du procédé).
De préférence, le procédé de l'invention permet de préparer un diol répondant à la formule (I-1) suivante :

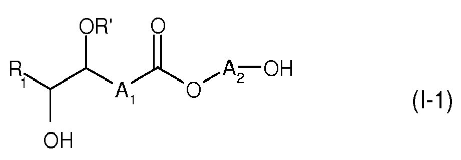

A₁, A₂, R₁ et R' étant tels que définis ci-dessus dans la formule (I).

Le diol de formule (I-1) correspond à un composé de formule (I) dans laquelle Y est un atome d'hydrogène. Ce diol comprend une fonction OH primaire (en bout de chaîne) et une fonction OH secondaire.

La présente invention concerne également un procédé de préparation d'un diol tel que défini ci-dessus, ledit diol répondant à la formule (I-2) suivante : 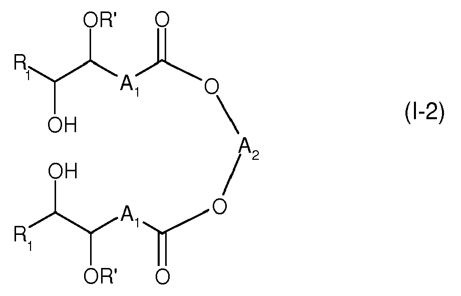 A₁, A₂, R₁ et R' étant tels que définis ci-dessus dans la formule (I).

Le diol de formule (I-2) correspond à un composé de formule (I) dans laquelle Y est un groupe de formule (A). Ce diol comprend deux fonctions OH secondaires.

Selon un mode de réalisation préféré, l'étape a) du procédé de l'invention est effectuée en présence d'un catalyseur choisi dans le groupe constitué de l'oxyde de magnésium, de l'acétate de zinc et du méthanolate de sodium.

De préférence, ladite étape a) est effectuée à une température comprise de 150 à 200°C sous flux d'azote. Cette gamme de température est choisie en fonction de la nature du catalyseur utilisé. Par exemple, si la température est supérieure à 200°C, le catalyseur est dégradé.

Selon un mode de réalisation préféré, cette étape a) telle que définie ci-dessus est effectuée sans solvant, ce qui est très avantageux en termes d'écologie.

La présente invention concerne également un procédé de préparation d'un diol tel que défini ci-dessus, caractérisé en ce que le produit de formule (IV) obtenu à l'issue de l'étape a) est sous la forme d'un mélange de monoesters et de diesters, les monoesters répondant à la formule (IV-1) suivante : 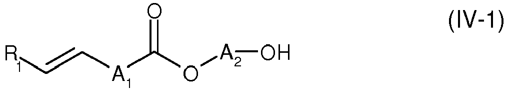 et les diesters répondant à la formule (IV-2) suivante : 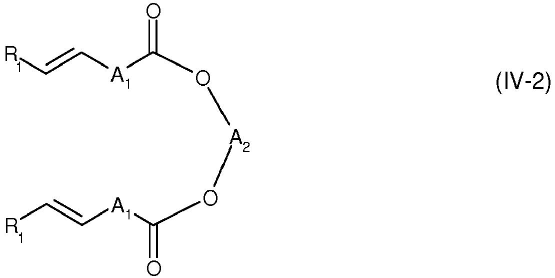 A₁, A₂ et R₁ étant tels que définis ci-dessus dans la formule (I).

Le monoester de formule (IV-1) correspond à un composé de formule (IV) dans laquelle Y₁ est H et le diester de formule (IV-2) correspond à un composé de formule (IV) dans laquelle Y₁ est un groupe de formule (A₁).

Dans le cadre du procédé de la présente invention, si le diol, à savoir le composé de formule (III), est utilisé en grand excès (par exemple à un ratio molaire de 100)(ratio entre le nombre de moles du diol (III) et le nombre de moles du composé de formule (II)), on obtient jusqu'à 95% en poids de monoester (composé de formule (IV-1)), tandis que lorsque le diol est utilisé en défaut par rapport au composé de formule (II) (par exemple avec un ratio molaire de 0,5), on obtient jusqu'à 85% en poids de diester (composé de formule (IV-2)). Le procédé de l'invention permet donc de contrôler la composition du composé obtenu (monoester, diester ou mélange) par un choix approprié des quantités des produits de départ.

La présente invention concerne également un procédé de préparation d'un diol tel que défini ci-dessus, caractérisé en ce que le produit de formule (V) obtenu à l'issue de l'étape b) est sous la forme d'un mélange de monoesters et de diesters, les monoesters répondant à la formule (V-1) suivante : 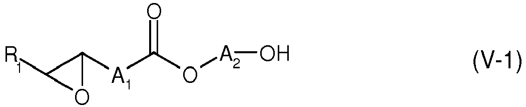 et les diesters répondant à la formule (V-2) suivante : 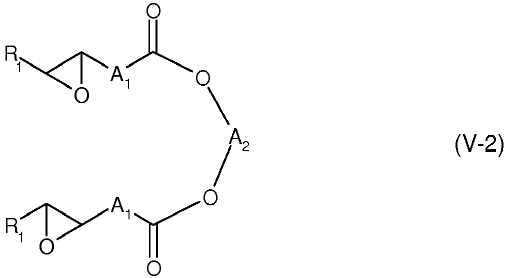 A₁, A₂ et R₁ étant tels que définis ci-dessus dans la formule (I).

Le monoester de formule (V-1) correspond à un composé de formule (V) dans laquelle Y₂ est H et le diester de formule (V-2) correspond à un composé de formule (V) dans laquelle Y₂ est un groupe de formule (A₂).

Le procédé susmentionné selon l'invention peut également comprendre une étape intermédiaire entre l'étape a) et l'étape b) qui consiste à purifier les composés de formule (IV), notamment sur une colonne de silice ou par distillation sous vide poussé.

Par exemple, le monoester de formule (IV-1) est purifié en utilisant un mélange 80/10/10 heptane/acétone/éther de pétrole et le diester de formule (IV-2) est purifié en utilisant un mélange 80/20 heptane/éther de pétrole.

Selon un mode de réalisation préféré, l'étape b) du procédé de l'invention est effectuée en présence d'un peracide, cette étape étant notamment effectuée sous vide à 200°C pendant moins de 3 minutes.

Parmi les peracides, on peut notamment citer l'acide métachloroperbenzoïque (m-CPBA) et le peracide magnésium monoperoxyphthalate hexahydrate (MMPP).

La présente invention concerne également un procédé de préparation d'un diol tel que défini ci-dessus, dans lequel, lorsque le composé de formule (IV) est un composé dans lequel Y₂ est un groupe de formule (A₂), l'étape b) d'époxydation est effectuée en présence d'H₂O₂ et d'acide formique ou en présence d'H₂O₂ et d'acide acétique.

Le procédé susmentionné selon l'invention peut également comprendre une étape intermédiaire entre l'étape b) et l'étape c) qui consiste à purifier les composés de formule (V), notamment sur une colonne de silice.

Toutefois, cette étape de purification est facultative étant donné que les composés de formule (V) peuvent également être utilisés directement sans être purifiés.

Par exemple, le monoester de formule (V-1) est purifié en utilisant un mélange 60/40 toluène/acétate d'éthyle et le diester de formule (V-2) est purifié en utilisant un mélange 95/5 toluène/acétate d'éthyle.

Selon un mode de réalisation préféré, le procédé de préparation selon l'invention est caractérisé en ce que l'étape c) est effectuée en présence d'un catalyseur choisi dans le groupe constitué d'un catalyseur acide de type résine échangeuse d'ions protonique, notamment avec des fonctions acide sulfonique (résine Amberlyst® 15 Dry), des catalyseurs hétérogènes, de l'acide para-tolènesulfonique (APTS) ou de l'acide méthanesulfonique (AMS), à une température comprise de 20°C à 120°C, et de préférence à 70°C.

Le procédé susmentionné selon l'invention peut également comprendre une étape complémentaire consistant, à l'issue de l'étape c), à purifier les composés de formule (I), notamment sur une colonne de silice.

Toutefois, cette étape de purification est facultative étant donné que les composés de formule (I) peuvent également être utilisés directement sans être purifiés.

Par exemple, les composés de formule (I) sont purifiés en utilisant un mélange 40/60 toluène/acétate d'éthyle.

### Description détaillée des étapes du procédé de l'invention

### 1. Etape a) : réaction de transestérification des composés de formule (II') ou (II)

Dans le cadre du procédé de l'invention, cette transestérification s'effectue de préférence à partir d'un ester d'alcool léger (notamment méthanol ou éthanol...) de tournesol oléique (composé de formule (II) ou (II')) et d'un diol (composé de formule (III) ou (III')) en présence notamment d'oxyde de magnésium comme catalyseur. Plusieurs synthèses sont réalisées avec différents diols afin de moduler les propriétés des monomères et donc des polymères en résultant. Des transestérifications ont donc été réalisées à partir de propanediol, d'hexanediol, de butanediol et de poly(oxyde d'éthylène)hydroxytéléchélique.

La réaction se déroule entre 150°C et 200°C sous flux d'azote. L'avancement de la réaction est suivi par différentes analyses et notamment la RMN (disparition du singulet du groupement méthyle). Selon les conditions de réaction, deux produits sont obtenus :
Si le diol utilisé est placé en grand excès, on obtient en majorité au moins 80%, voire 95%, de monoesters (ou dérivés de monoglycérides) possédant un groupement hydroxyle terminal. Cet alcool en bout de chaîne apporte alors une première fonctionnalité au monomère.

A l'inverse, si le diol est volontairement introduit en défaut, on obtient en majorité au moins 60%, voire 85%, de diesters (ou dérivés de diglycérides). Ce second précurseur possède alors exactement deux doubles liaisons par lesquelles seront introduits les groupements hydroxyles, permettant l'accès à des monomères de fonctionnalité égale à deux.

| | **Temps de réaction** | | **Rendement** | |
|---|---|---|---|---|
| **Alcools utilisés (II) ou (II')** | Synthèse de monoesters | Synthèse de diesters | Synthèse de monoesters | Synthèse de diesters |
| Propanediol | 10h | 15h | 80% | 62% |
| Hexanediol | 10h | 15h | 80% | 60% |
| Polyoxyde d'éthylène (M_{w}=300 g/mol) | 15h | 20h | 75% | 59% |
| Polyoxyde d'éthylène (M_{w}=600 g/mol) | 15h | 20h | 75% | 59% |

L'étape a) (avec R₁ = C₆H₁₃ ; A₁ = C₉H₁₈ ; R₂ = CH₃ ; A₂ = R) peut ainsi notamment être représentée par le schéma suivant :

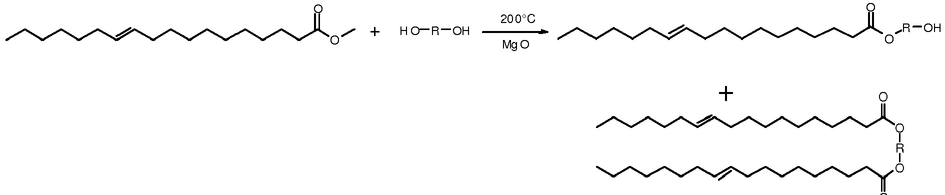

Les synthons préparés (correspondant aux composés de formule (IV)) sont par exemple purifiés sur colonne de silice avec un mélange 80/10/10 heptane/acétone/éther de pétrole pour le monoester et un mélange 80/20 heptane /éther de pétrole pour le diester. Les rendements après purification sont donnés dans le tableau ci-dessus.

A la fin de cette première étape on dispose de deux précurseurs : le premier est un monoester (composé de formule (IV-1)) possédant un groupement hydroxyle terminal et une double liaison sur la chaîne ; le second est un diester (composé de formule (IV-2)) possédant exactement deux doubles liaisons afin d'obtenir par la suite un polyol symétrique contenant deux groupements hydroxyles. La voie de synthèse mise en jeu est « propre » car elle fait appel à une catalyse hétérogène (oxyde de magnésium) et la synthèse se déroule sans solvant. La purification industrielle peut se faire par distillation sous vide poussé.

### 2. Etape b) : époxvdation des dérivés de monoesters et diesters obtenus après transestérification

L'époxydation de corps gras possédant un alcool primaire en bout de chaîne par des *pera*cides formés *in situ* n'a jamais été traitée et ne peut pas être réalisée dans les mêmes conditions que celles décrites précédemment. La réaction d'époxydation est en effet parasitée par une réaction secondaire d'oxydation de l'alcool terminal du monoester de tournesol oléique pour former un acide carboxylique. Le réactif étant consommé par cette réaction secondaire, l'époxydation ne se réalise pas.

Ainsi, une réaction secondaire d'estérification entre le catalyseur (acide carboxylique) et l'alcool terminal du monoester se produit selon le schéma suivant (cas spécifique avec R₁ = C₈H₁₇ et A₁ = C₇H₁₄) :

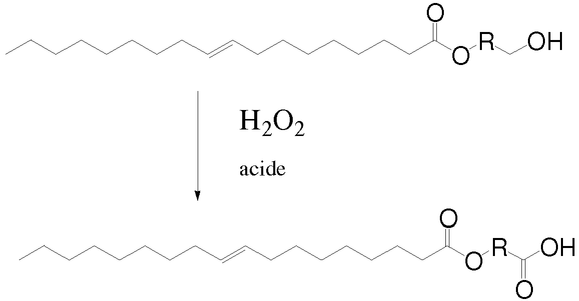

La diminution de la quantité de catalyseur ou de la température afin de défavoriser la réaction parasite entraîne néanmoins une augmentation significative du temps de réaction, et en parallèle un début d'ouverture des ponts époxyde formés, fragiles en milieu acide à 70°C. Une autre stratégie d'époxydation sans l'utilisation d'acide carboxylique réactif a donc été mise au point par les inventeurs.

La méthode mise en oeuvre dans le cadre du procédé de l'invention pour l'époxydation de monoester à groupement hydroxyle terminal (composés de formule (IV-1)) réside dans l'utilisation d'un peracide déjà formé et commercialisable, à savoir notamment l'acide métachloroperbenzoïque (m-CPBA), et évite donc l'utilisation de métaux potentiellement toxiques.

Le mécanisme de cette réaction peut être représentée selon le schéma ci-après :

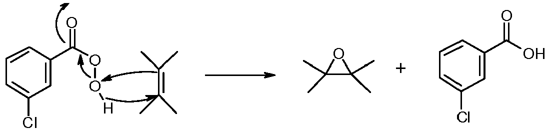

Dans le cadre de l'invention, l'époxydation a été réalisée avec des peracides (mCPBA, MMPP...). La réaction peut être suivie par RMN ; la disparition des doublets des protons de la double liaison à 5,2 ppm ainsi que l'apparition d'un pic large à 2,8 ppm permettent de suivre l'avancement de la réaction. La conversion des doubles liaisons est totale au bout de 3h. L'excès de m-CPBA est réduit en acide carboxylique correspondant avec une solution saturée de sulfate de sodium. La phase organique est extraite avec du dichlorométhane puis l'acide carboxylique résiduel est transformé en chlorobenzoate de sodium (soluble dans l'eau) grâce à deux lavages avec une solution saturée de bicarbonate de soude.

Les diesters (composés de formule (IV-2)), ne possédant pas de groupements hydroxyles, peuvent être époxydés en utilisant le protocole classique (H₂O₂ + acide formique) ou en utilisant un peracide comme indiqué ci-dessus, par exemple l'acide métachloroperbenzoique.

Les synthons obtenus sont tous purifiés sur colonne de silice avec un mélange 60/40 toluène/acétate d'éthyle pour le monoester époxydé et un mélange 95/5 toluène/acétate d'éthyle pour le diester époxydé.

### 3. Etape c) : ouverture des époxydes des monoesters et diesters époxvdés

### par des alcools

Dans le cadre de la présente invention, le but est d'introduire des groupements hydroxyles sur des monoesters possédant déjà un alcool primaire terminal par ouverture des époxydes par un alcool.

A 110°C et sous catalyse acide (acide p-toluènesulfonique), la transestérification est favorisée par rapport à l'ouverture de l'époxyde. En effet l'alcool primaire en bout de chaîne entre en jeu dans ces réactions secondaires.

On observe par exemple la formation de couplages lors de l'ouverture de l'epoxide notamment selon le schéma suivant :

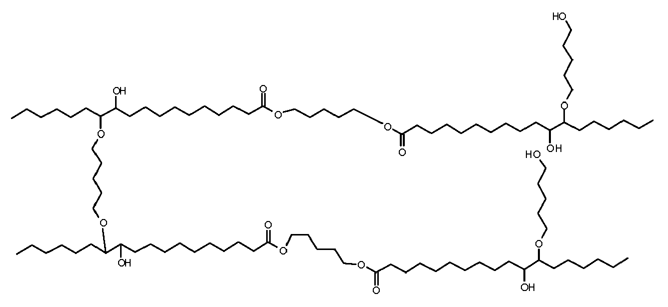

Des essais réalisés à plus basse température montrent que les couplages sont réduits mais l'ouverture de l'époxyde est considérablement ralentie. Beaucoup d'ouvertures d'époxydes utilisent des catalyseurs acides homogènes (US 4 508 853 ; Gruber et al., Fett Wissenschaft Technologie, 1987, 4:147-151 ; EP 0 260 499 ; US 4 742 087 ; DE 4 232 167 ; Guo et al., J. Polym. Sci. A: Polym. Chem., 2000, 38: 3900-3910 ; US 6 433 121 ; US 6 573 354 ; Zlatanic et al., J. Polym. Sci. B: Polym. Physics, 2004, 42: 809-819; US 20070232816) ou des amines (DE 4 238 215).

Le procédé de la présente invention consiste à utiliser un catalyseur spécifique à l'ouverture de l'époxyde et opérationnel à des températures plus basses afin de ne pas déclencher un début de transestérification.

Les conditions de réactions mises en oeuvre pour l'étape c) du procédé de l'invention sont plus douces qu'avec une catalyse homogène classique (70°C au lieu de 110°C) et la synthèse se fait sans solvant. Ainsi, les mono-esters et di-esters époxydés sont mélangés à un grand excès d'alcool en présence de 4% en masse de résine Amberlyst 15 Dry. Le milieu est chauffé à 70°C pendant 15h. L'avancement de la réaction est suivi par RMN grâce à la disparition des pics de l'époxyde à 2,8 ppm. Le mélange est ensuite filtré afin de récupérer le catalyseur.

Les synthons alcoolisés sont ensuite purifiés sur colonne de silice avec un mélange toluène/acétate d'éthyle 40/60 pour les monoesters et diesters hydroxylés.

Il est décrit un composé répondant à la formule générale (I") suivante : dans laquelle : 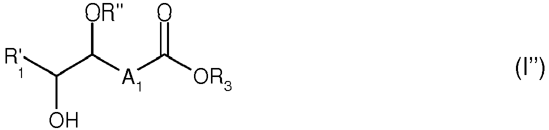
- R'₁ représente H ou un groupe alkyle, linéaire ou ramifié, comprenant de 2 à 14 atomes de carbone, ledit groupe alkyle pouvant éventuellement être substitué par un ou plusieurs groupes ORₐ, Rₐ représentant H ou un groupe R' tel que défini ci-dessous,
- A₁ représente un radical alkylène divalent linéaire ou ramifié, comprenant de 2 à 14 atomes de carbone,
- R" représente :
   - un groupe alkyle R', linéaire ou ramifié, comprenant de 1 à 18 atomes de carbone, ou
   - un groupe de formule -A₂-OH, A₂ représentant un radical alkylène divalent linéaire ou ramifié, comprenant de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
   A₂ représentant de préférence un radical de formule -CH₂-A₃-CH₂-, A₃ représentant un groupe de formule -(CH₂OCH₂)ₙ-, n représentant un nombre entier compris de 1 à 100, et de préférence égal à 6, 13 ou 45, ou un radical phénylène, et
- R₃ représente :
   - un groupe alkyle R₂, linéaire ou ramifié, comprenant de 1 à 10, de préférence de 1 à 6, atomes de carbone, ou
   - un groupe de formule -A₂-O-Y', A₂ étant tel que défini ci-dessus et Y' représentant un atome d'hydrogène ou un groupe de formule (A') 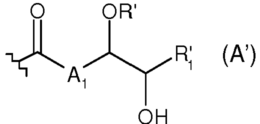
      A₁, R' et R'₁ étant tels que définis ci-dessus dans la formule (I"),
      étant entendu que lorsque R" est un groupe R' alors R₃ représente un groupe de formule -A₂-O-Y', et que lorsque R" est un groupe -A₂-OH alors R₃ représente un groupe R₂.

Il est décrit un composé répondant à la formule générale (I"-1) suivante : dans laquelle : 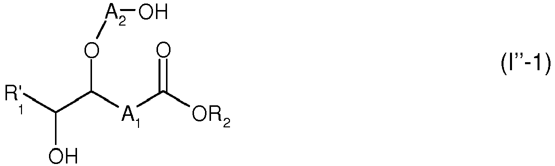
- R'₁ représente H ou un groupe alkyle, linéaire ou ramifié, comprenant de 2 à 14 atomes de carbone, ledit groupe alkyle pouvant éventuellement être substitué par un ou plusieurs groupes ORₐ, Rₐ représentant H ou un groupe R' tel que défini ci-dessous,
- A₁ représente un radical alkylène divalent linéaire ou ramifié, comprenant de 2 à 14 atomes de carbone,
- A₂ représente un radical alkylène divalent linéaire ou ramifié, comprenant de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
   A₂ représentant de préférence un radical de formule -CH₂-A₃-CH₂-, A₃ représentant un groupe de formule -(CH₂OCH₂)ₙ-, n représentant un nombre entier compris de 1 à 100, et de préférence égal à 6, 13 ou 45, ou un radical phénylène, et
- R₂ représente un groupe alkyle, linéaire ou ramifié, comprenant de 1 à 10, de préférence de 1 à 6, atomes de carbone.

Il est décrit un composé répondant à la formule générale (I') suivante : dans laquelle : 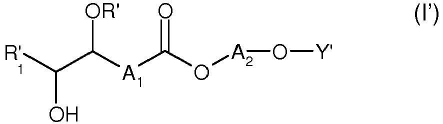
R'₁ représente H ou un groupe alkyle, linéaire ou ramifié, comprenant de 2 à 14 atomes de carbone, ledit groupe alkyle pouvant éventuellement être substitué par un ou plusieurs groupes ORₐ, Rₐ représentant H ou un groupe R' tel que défini ci-dessous,
R' représente un groupe alkyle, linéaire ou ramifié, comprenant de 1 à 18 atomes de carbone,
A₁ représente un radical alkylène divalent linéaire ou ramifié, comprenant de 2 à 14 atomes de carbone,
A₂ représente un radical alkylène divalent linéaire ou ramifié, comprenant de 1 à 10 atomes de carbone, le cas échéant comprenant un ou plusieurs substituants, notamment choisis dans le groupe constitué du radical phénylène et du radical de formule -(CH₂OCH₂)ₙ-, n représentant un nombre entier compris de 1 à 100, et de préférence égal à 6, 13 ou 45,
Y' représente un atome d'hydrogène ou un groupe de formule (A') 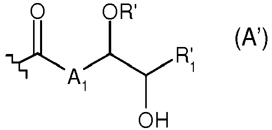
A₁, R' et R'₁ étant tels que définis ci-dessus dans la formule (I').

Parmi les composés préférés susmentionnés, on peut notamment citer les composés de formule (I) suivante : 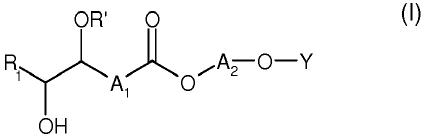 dans laquelle :
R₁ représente un groupe alkyle, linéaire ou ramifié, comprenant de 2 à 14 atomes de carbone, et
R', A₁ et A₂ sont tels que définis ci-dessus dans la formule (I'), et
Y représente un atome d'hydrogène ou un groupe de formule (A) 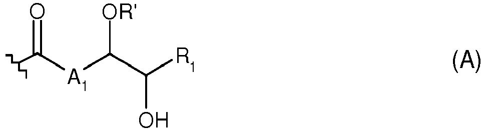
A₁, R' et R₁ étant tels que définis ci-dessus dans la formule (I).

On peut également citer d'autres composés préférés selon la présente invention, répondant à la formule générale (I-1) suivante : dans laquelle : 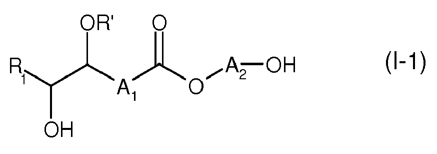 R₁, R', A₁ et A₂ sont tels que définis ci-dessus dans les formules (I') et (I).

Des composés répondant à la formule générale (I"-1-1) suivante sont décrits : dans laquelle : 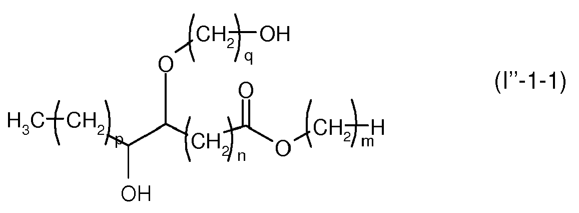 m, n_{,} p et q sont des nombres entiers compris de 1 à 18, m étant de préférence égal à 2.

De préférence, dans la formule (I"-1-1), q est égal à 4.

La présente invention concerne également des composés répondant à la formule générale (I-1-1) suivante : 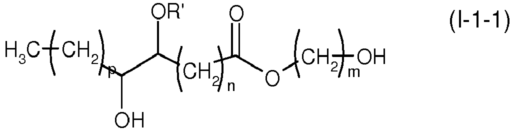 dans laquelle :
m, n et p sont des nombres entiers compris de 1 à 18,
et R' est tel que défini ci-dessus dans la formule (I').

La présente invention concerne également des composés répondant à la formule générale (I-1-2) suivante :

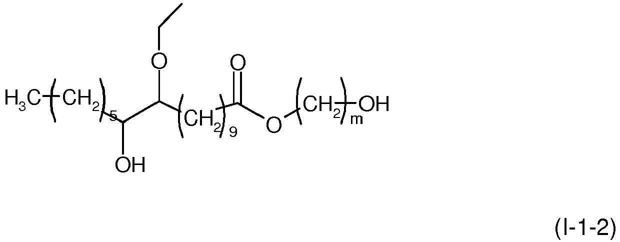

Une autre famille de composés préférés de l'invention est constituée des composés répondant à la formule générale (I-2) suivante : dans laquelle : 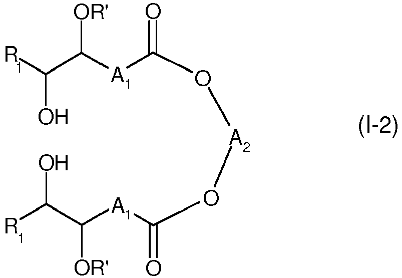 R₁, R', A₁ et A₂ sont tels que définis ci-dessus dans les formules (I') et (I).

Une autre famille de composés préférés de l'invention est constituée des composés répondant à la formule générale (I-2-1) suivante : dans laquelle : 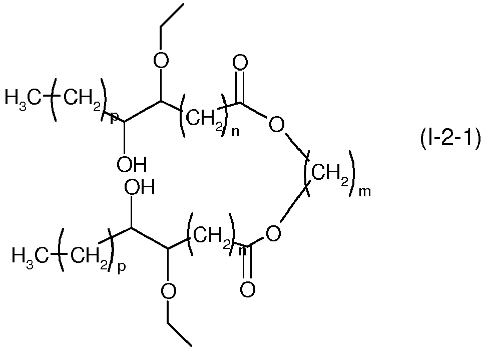
m, n et p sont des nombres entiers compris de 1 à 18,
et R' est tel que défini ci-dessus dans la formule (I').

Une autre famille de composés préférés de l'invention est constituée des composés répondant à la formule générale (I-2-2) suivante : 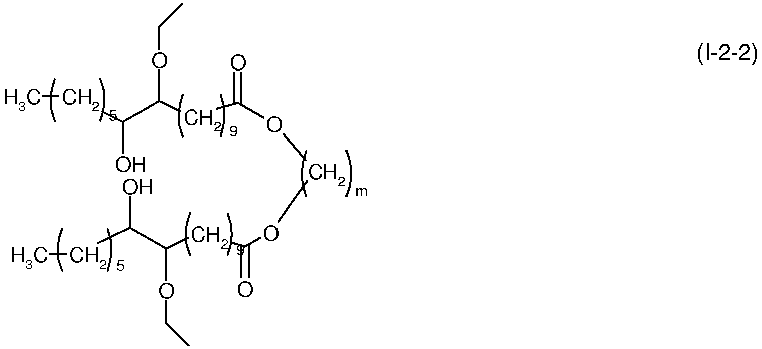 m étant tel que défini ci-dessus.

Parmi les polyols préférés de l'invention, on peut notamment citer les deux composés spécifiques suivants :
- un diol (8) dérivé d'huile de tournesol : 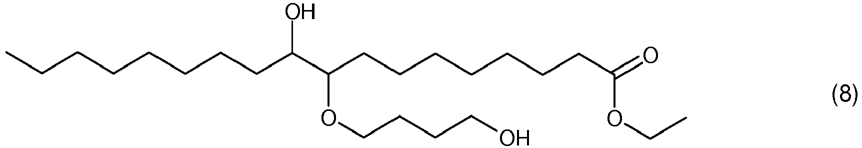
- un diol (11) dérivé d'huile de colza :

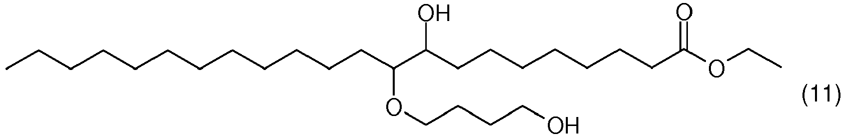

Les polyols selon la présente invention, notamment les diols, ont la spécificité d'être bien définis, avec deux groupements hydroxyles primaires ou secondaires. Les dérivés de diesters sont originaux en raison de leur symétrie et l'alcool utilisé pour la transestérification permet de varier la structure des synthons et ainsi les propriétés des polymères résultants.

Les diols obtenus par ces différentes méthodes peuvent alors être utilisés, entre autres, comme monomères. Leur pureté permet d'optimiser les propriétés des polymères obtenus.

Ainsi, des polyuréthanes ont ensuite été synthétisés par polymérisation en masse de ces polyols avec l'IPDI (ou par exemple également avec du MDI, du HMDI ou de l'HDI), à 60°C en présence de dibutyl dilaurate d'étain. La formation des polyuréthanes est confirmée par FTIR avec la disparition de la bande de vibration de l'isocyanate. La chromatographie d'exclusion stérique confirme des masses molaires comprises entre 14 000 et 50 000 g/mol. Ces monomères di-OH peuvent également être utilisés pour la synthèse d'autres polymères tels que des polyesters, polyéthers, polycarbonates, etc.

Les composés polyols selon la présente invention de formule (I) sont notamment utilisés pour réagir avec des polyisocyanates pour former des polyuréthanes.

Ainsi, ces composés peuvent être utilisés pour la préparation de mousses rigides, d'isolants électriques, de revêtements, d'adhésifs, de mousses flexibles (notamment dans le domaine de l'ameublement ou de l'automobile) ou de semelles de chaussures.

Plus exactement, les polyols selon la présente invention sont utilisés pour la préparation de mousses rigides en les faisant réagir avec des polyisocyanates en présence d'un catalyseur et d'un agent moussant (auxquels on peut également ajouter des surfactants, des colorants, des antioxydants, des conservateurs, des agents plastifiants, des agents de réticulation, des ignifuges, etc.).

De préférence, on peut préparer une telle mousse rigide en faisant réagir ensemble les constituants suivants : 60 g de polyisocyanate, 40 g de polyol, 1,2 g d'eau (agent moussant), 0,1-0,4 g de catalyseur et 1-4 g de surfactant.

Plus exactement, les polyols selon la présente invention sont utilisés pour la préparation d'isolants électriques en les faisant réagir avec des polyisocyanates en présence d'un agent anti-mousse et d'un agent séchant.

De préférence, un tel isolant électrique peut être préparé en faisant réagir ensemble 60 g de polyol, 29 g de polyisocyanate, 0,6 g d'agent anti-mousse et 3 g d'agent séchant, et éventuellement 60 g de charges (silice).

Plus exactement, les polyols selon la présente invention sont utilisés pour la préparation de revêtements en les faisant réagir avec des polyisocyanates. Par exemple, des revêtements sont préparés en utilisant les polyols et les polyisocyanate purs, ou en utilisant les polyols et les polyisocyanate avec solvants (on peut également ajouter des colorants, des pigments, des charges, des additifs rhéologiques, des antioxydants, des bactéricides, des fongicides, des inhibiteurs de corrosion, des catalyseurs ou des stabilisateurs UV).

Pour la préparation d'adhésifs selon la présente invention, on prévoit également d'utiliser les polyols de l'invention purs avec des polyisocyanates purs.

En ce qui concerne les mousses flexibles, de préférence, on utilise 60 g de polyol selon l'invention, 100 g d'isocyanate, 4.5 g d'eau (agent moussant), 0,12 g de catalyseur 1, 0,38 g de catalyseur 2 et 3 g de surfactant.

Enfin, une formulation spécifique selon l'invention pour la préparation de semelles de chaussures comprend 59 g d'isocyanate, 94,5 g de polyol selon l'invention, 4,1 g d'éthylène glycol et 1,4 g de catalyseur.

Les composés intermédiaires répondant à l'une des formules suivantes sont décrits :

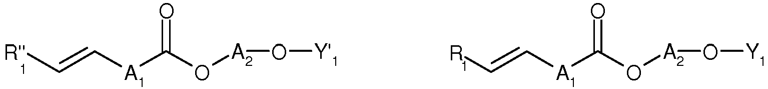

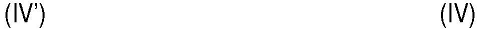

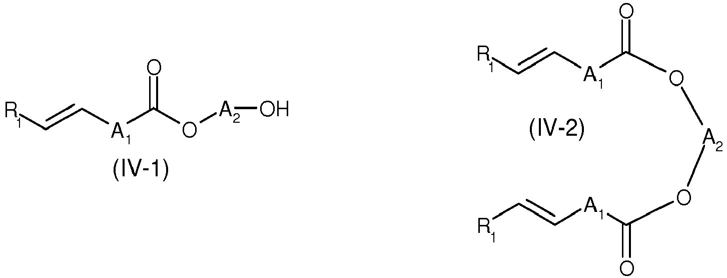

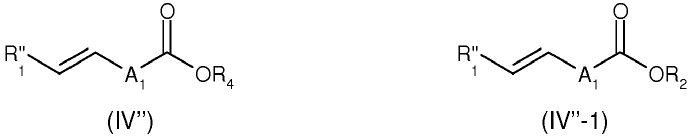

R"₁, A₁, A₂, Y₁, Y'₁, R₂, R₄ et R₁ étant tels que définis ci-dessus dans les formules (I), (I') et (I").

Les composés intermédiaires répondant à l'une des formules suivantes sont décrits :

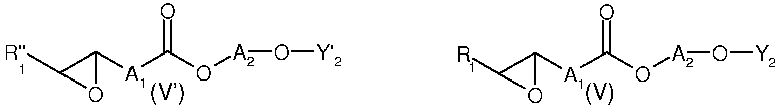

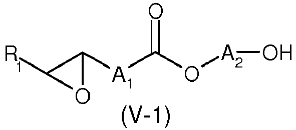

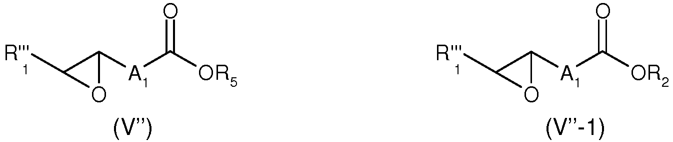

R"₁, A₁, A₂, Y₂, Y'₂, R₅, R₂ et R₁ étant tels que définis ci-dessus dans les formules (I) et (I').

L'invention concerne des composés intermédiaires de formule (V-2) suivante :

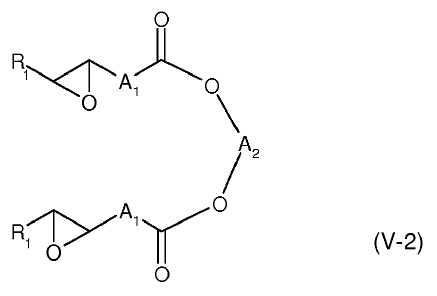

A₁, A₂ et R₁ étant tels que définis ci-dessus dans la formule (I).

Une famille particulièrement préférée de composés intermédiaires est décrite et constituée de composés répondant à la formule suivante (V-3) :

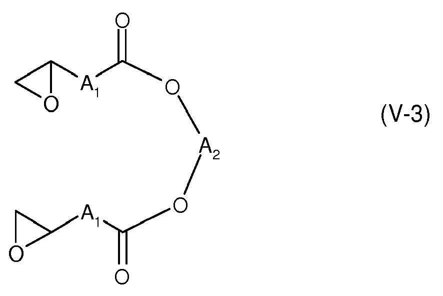

A₁ et A₂ étant tels que définis ci-dessus dans la formule R"₁ et A₁ représentant de préférence un groupe C₇H₁₄.

La présente invention concerne donc également la synthèse de précurseurs bis-époxydes, notamment pour résines époxy.

Ces précurseurs bis-époxydes possèdent deux groupements époxydes terminaux et se rapprochent de la structure du diglycidyl éther de bisphénol A (DGEBA). Le DGEBA est très utilisé comme précurseur dans la synthèse de résine époxy, par réaction de condensation avec des diamines. Cependant le DGEBA est en voie d'interdiction à cause de la toxicité de son bisphénol A. Les bis-époxydes selon l'invention de formule (V-3) susmentionnée s'avèrent être une alternative à l'utilisation du DGEBA.

La littérature décrit déjà l'utilisation d'huile végétale dans la formulation de résines époxy : huile de tournesol époxydée (Jiang Zhu et al., Journal of Applied Polymer Science, 2004, 91, 3513-3518), huile de ricin époxydée (Park et al., Macromolecular Chemistry and Physics, 2004, 205, 2048-2054) ou huile de soja carbonatée (Parzuchowski et al., Journal of Applied Polymer Science, 2006, 102, 2904-2914). Néanmoins ces huiles ne remplacent qu'une faible proportion de DGEBA en raison de leur faible réactivité. Les résines époxy obtenues présentent des propriétés mécaniques équivalentes voire supérieures aux résines époxy commerciales issues du pétrole. Pour l'instant, un maximum de 30% d'huile époxydée a été incorporée aux résines commerciales. Le mélange d'huile de lin époxydée, de diglycidyl ether de bisphenol F (DGEBF), et d'un adjuvant anhydride permet d'obtenir des résines contenant jusqu'à 70% d'huile de lin époxydée (Miyagawa et al., Marcomoecular Materials and Engineering, 2004, 289, 629-635).

La synthèse de résines époxy à partir de 100% d'huile végétale n'est pas possible en raison de la faible réactivité des groupements époxydes internes à la chaîne. La voie de synthèse proposée dans le cadre de la présente invention permet l'obtention de précurseurs possédant deux groupements époxydes terminaux, ce qui augmente la réactivité vis-à-vis des amines et permet la synthèse de résines époxy à partir de 100% d'huile végétale.

Le procédé de préparation des composés de formule (V-3) peut être représenté par le schéma ci-après :

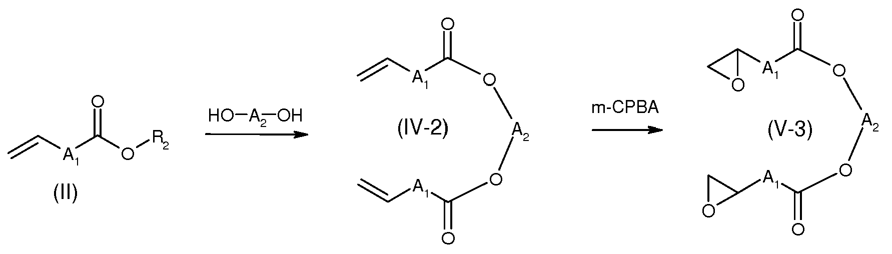

De préférence, A₁ représente un radical C₇H₁₄.

La présente invention concerne également les polymères de type polyuréthane tels qu'obtenus par polymérisation des polyols de la présente invention, notamment de formule (I), avec des (poly)isocyanates.

Elle concerne également les polymères de type polyesters tels qu'obtenus par polymérisation des polyols de la présente invention, notamment de formule (I).

### PARTIE EXPÉRIMENTALE

### Exemple 1 : Préparation d'esters éthyliques d'huile de tournesol oléique

Cet exemple concerne la préparation du composé (1) de formule suivante :

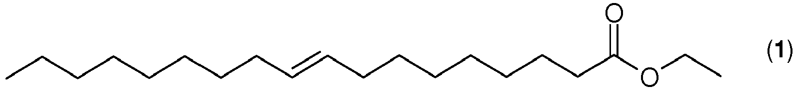

Il s'agit d'un composé de formule (II) dans laquelle R₁ représente un groupe alkyle comprenant 8 atomes de carbone, A₁ représente un radical alkylène comprenant 7 atomes de carbone et R₂ représente un groupe éthyle.

Le produit de départ est l'huile de tournesol oléique (HTO) de formule :

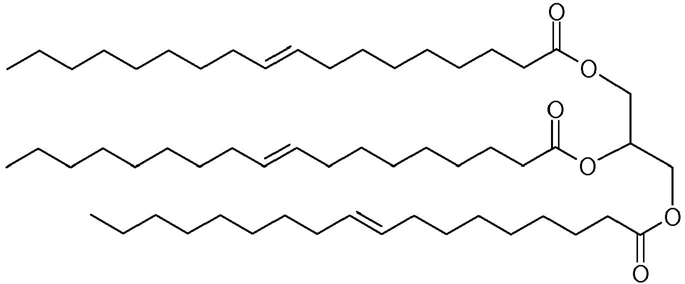

On a introduit dans un réacteur double enveloppe 604,8 g d'huile de tournesol oléique (HTO)(ITERG, M = 884,82 g.mol⁻¹ - teneur en eau = 0,35% en poids) avec 188,2 g d'éthanol absolu (JT Baker - M = 46,07 g.mol⁻¹). L'ensemble est mélangé sous agitation à 650 tr.min⁻¹ et chauffé à 65°C. On a ensuite ajouté 6,7211 g de MeONa (Aldrich - M = 54,02 g.mol⁻¹) dans le réacteur et on a alors constaté un changement de couleur du produit et l'apparition d'un trouble instantané. L'ensemble a ensuite été laissé réagir pendant 1 heure à 70°C.

Le mélange réactionnel résultant a ensuite été transvasé dans une ampoule à décanter afin d'éliminer le glycérol et évaporer l'éthanol. On a ensuite effectué une neutralisation avec quelques gouttes de HCI puis un lavage à l'eau jusqu'à neutralité. Enfin, l'eau résiduelle a été distillée au rotavapor.

On a ainsi obtenu 532,1 g d'ester éthylique d'huile de tournesol de formule (1) susmentionnée avec une teneur en eau de 0,35% en poids.

D'après la caractérisation par chromatographie en phase gazeuse effectuée, on a obtenu une composition comprenant 98,2% en poids d'ester éthylique.

### Exemple 1bis : Préparation d'esters éthyliques d'huile de tournesol oléique

Cet exemple concerne la préparation du composé (1) de l'exemple 1 susmentionné à partir d'huile de tournesol oléique (HTO).

On a introduit dans un réacteur double enveloppe 502,8 g d'huile de tournesol oléique (HTO)(ITERG) avec 161,5 g d'éthanol absolu (JT Baker - M = 46,07 g.mol⁻¹). L'ensemble est mélangé sous agitation à 650 tr.min⁻¹ et chauffé à 65°C. On a ensuite ajouté 5,5880 g de MeONa (Aldrich - M = 54,02 g.mol⁻¹) dans le réacteur et on a alors constaté un changement de couleur du produit et l'apparition d'un trouble instantané. L'ensemble a ensuite été laissé réagir pendant 5 heures à 70°C.

Le mélange réactionnel résultant a ensuite été transvasé dans une ampoule à décanter afin d'éliminer le glycérol et évaporer l'éthanol. On a ensuite effectué une neutralisation avec quelques gouttes de HCI puis un lavage à l'eau jusqu'à neutralité. Enfin, l'eau résiduelle a été distillée au rotavapor.

On a ainsi obtenu 455,2 g d'ester éthylique d'huile de tournesol de formule (1) susmentionnée avec une teneur en eau de 0,29% en poids.

D'après la caractérisation par chromatographie en phase gazeuse effectuée, on a obtenu une composition comprenant 97,4% en poids d'ester éthylique.

### Exemple 2 : Préparation d'esters de butanediol d'huile de tournesol oléique

Cet exemple concerne la préparation du composé (2) de formule suivante :

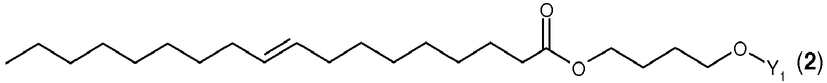

Il s'agit d'un composé de formule (IV) dans laquelle R₁ représente un groupe alkyle comprenant 8 atomes de carbone, A₁ représente un radical alkylène comprenant 7 atomes de carbone, A₂ représente un radical butylène et Y₁ représente H ou un groupe de formule (A₁) telle que définie ci-dessus.

Le produit de départ est le composé (1) tel qu'obtenu dans l'exemple 1.

On a introduit dans un réacteur (500 mL) 301,5 g de composé (1) (EEHTO) avec 43,1 g (0,5 mol) de 1,4-butanediol (Aldrich)(composé de formule (III) avec A₂ = butylène). L'ensemble est chauffé à 65°C. On a ensuite ajouté 3,3602 g de MeONa (Aldrich) dans le réacteur et on a alors constaté un changement de couleur du produit (jaune opaque). L'ensemble est ensuite laissé réagir pendant 6 heures à 70-75°C sous agitation (650 tr.min⁻¹) à une pression de 800 à 300 mbar.

On a ensuite effectué une neutralisation avec quelques gouttes de HCI puis un lavage à l'eau pour éliminer les traces de butanediol jusqu'à neutralité. Enfin, l'eau résiduelle a été distillée au rotavapor.

On a ainsi obtenu 279 g d'ester de butanediol d'huile de tournesol de formule (2) susmentionnée avec une teneur en eau de 0,22% en poids. Le produit de formule (2) est sous la forme d'un liquide jaune limpide et présente un indice d'acide de 3,58%.

D'après la caractérisation par chromatographie en phase gazeuse effectuée, on a obtenu une composition comprenant :
- au bout de 1 heure : 70% en poids de diesters (composé (2) avec Y₁ = (A₁)), 11,1% en poids de monoesters (composé (2) avec Y₁ = H) et 18,9% en poids de composé (1).
- au bout de 7 heures : 74,5% en poids de diesters (composé (2) avec Y₁ = (A₁)), 12,5% en poids de monoesters (composé (2) avec Y₁ = H) et 16% en poids de composé (1).

Par ailleurs, des purifications complémentaires ont permis d'augmenter le rendement et notamment d'obtenir jusqu'à 85% en poids de diesters, et ce en effectuant une distillation des monoesters résiduels.

### Exemple 3 : Préparation d'esters de butanediol époxydés d'huile de tournesol oléique

Cet exemple concerne la préparation du composé (3) de formule suivante :

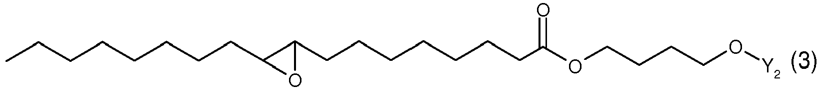

Il s'agit d'un composé de formule (V) dans laquelle R₁ représente un groupe alkyle comprenant 8 atomes de carbone, A₁ représente un radical alkylène comprenant 7 atomes de carbone, A₂ représente un radical butylène et Y₂ représente H ou un groupe de formule (A₂) telle que définie ci-dessus.

Le produit de départ est le composé (2) tel qu'obtenu dans l'exemple 2 présentant la composition suivante : 83,7% en poids de diesters, 8,80% en poids de monoesters et 7,50% en poids d'ester éthylique (composé (1)).

On a introduit dans un réacteur (250 mL) 79,7 g de composé (2) (esters de butanediol) avec 7 g (0,3 mol) d'acide formique HCOOH (BAKER) à 45°C pendant 1 heure à 500 tr.min⁻¹. On a ensuite additionné par une ampoule à brome au goutte à goutte de l'eau oxygénée pendant 10 minutes (36,7 g (2 moles) de H₂O₂ 50% (BAKER)). L'ensemble a ensuite été laissé réagir pendant 2 heures à 75°C sous agitation à 650 tr.min⁻¹. La réaction étant exothermique, le milieu a été refroidi avec un bain d'eau froide.

On a ensuite effectué un lavage à l'eau jusqu'à neutralité des eaux de lavage. Enfin, l'eau résiduelle a été distillée au rotavapor.

On a ainsi obtenu 79,3 g d'esters de butanediol époxydés d'huile de tournesol de formule (3) susmentionnée. Le produit de formule (3) est sous la forme d'un solide blanc à température ambiante et présente un indice d'acide de 1,95%.

D'après la caractérisation par chromatographie en phase gazeuse effectuée, on a obtenu une composition comprenant 86,8% en poids de diesters (composé (3) avec Y₂ = (A₂)), 7,3% en poids de monoesters (composé (3) avec Y₂ = H) et 5,9% en poids de composé (1).

### Exemple 4 : Préparation du diol (4)

Cet exemple concerne la préparation du composé **(4)** de formule suivante :

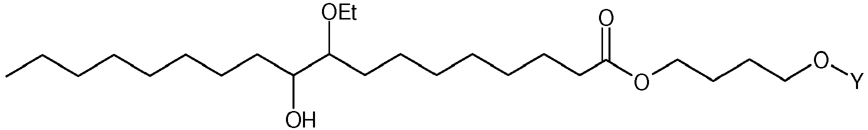

Il s'agit d'un composé de formule (I) dans laquelle R₁ représente un groupe alkyle comprenant 8 atomes de carbone, A₁ représente un radical alkylène comprenant 7 atomes de carbone, A₂ représente un radical butylène, R' représente un groupe éthyle et Y représente H ou un groupe de formule (A) telle que définie ci-dessus.

Le produit de départ est le composé (3) tel qu'obtenu dans l'exemple 3 présentant la composition suivante : 86,8% en poids de diesters, 7,3% en poids de monoesters et 5,9% en poids d'ester éthylique (composé (1)).

On a introduit dans un réacteur (250 mL) 60,2 g de composé (3) (esters époxydés de butanediol) avec 2,4 g (4% en poids) de résine Amberlyst (Aldrich) et 112,6 g (15 moles) d'éthanol absolu (BAKER). On a laissé l'ensemble réagir à 70°C pendant 4 heures à 500 tr.min⁻¹. On a ensuite filtré la résine sur Büchner et, enfin, l'éthanol résiduel a été distillé au rotavapor.

On a ainsi obtenu 53,6 g du polyol de formule (**4**) susmentionnée. Le produit de formule (3) est sous la forme d'un liquide jaune clair et présente un indice d'acide de 1,37%.

D'après la caractérisation par chromatographie en phase gazeuse effectuée, on a obtenu une composition comprenant moins de 0,5% en poids de butanediol, 82,0% en poids de diesters (composé (I) avec Y = (A)), 7,6% en poids de monoesters (composé (I) avec Y = H) et 10,4% en poids de composé (1).

Le composé (**4**) a été analysé par chromatographie IR et on a observé une bande OH à 3 461,76 cm⁻¹ et une bande alcool secondaire à 1 087,24 cm⁻¹.

### Exemple 5 : Préparation d'esters éthyliques d'huile de ricin

Cet exemple concerne la préparation du composé (**5**) de formule suivante :

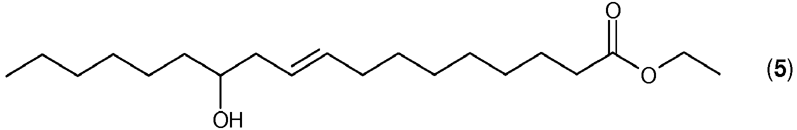

Il s'agit d'un composé de formule (II') dans laquelle R"₁ représente un groupe alkyle comprenant 8 atomes de carbone et substitué par un groupe OH (sur le carbone 7), A₁ représente un radical alkylène comprenant 7 atomes de carbone et R₂ représente un groupe éthyle.

Le produit de départ est l'huile de ricin de formule :

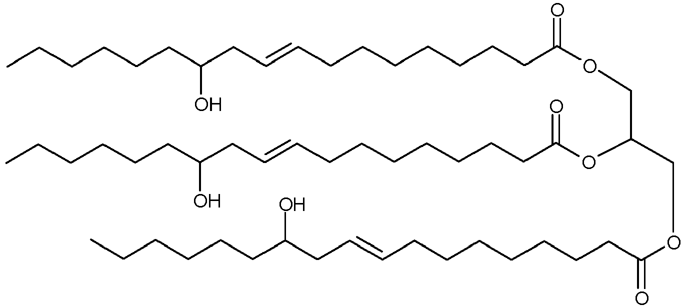

On a introduit dans un réacteur double enveloppe (de 1 litre) 402,4 g d'huile de ricin (ITERG, M = 928 g.mol⁻¹ - teneur en eau = 0,35% en poids) avec 405,4 g d'éthanol absolu (JT Baker - M = 46,07 g.mol⁻¹). L'ensemble est mélangé sous agitation à 650 tr.min⁻¹ et chauffé à 65°C. On a ensuite ajouté 4,5214 g de MeONa (Aldrich - M = 54,02 g.mol⁻¹) dans le réacteur et on a alors constaté un changement de couleur du produit et l'apparition d'un trouble instantané. L'ensemble est ensuite laissé réagir pendant 30 minutes à 50°C.

Le mélange réactionnel résultant a ensuite été transvasé dans une ampoule à décanter afin d'éliminer le glycérol et évaporer l'éthanol. On a ensuite effectué une neutralisation avec quelques gouttes de HCI puis un lavage à l'eau jusqu'à neutralité. Enfin, l'eau résiduelle a été distillée au rotavapor.

On a ainsi obtenu 360,2 g d'ester éthylique d'huile de ricin de formule (5) susmentionnée avec une teneur en eau de 0,23% en poids.

D'après la caractérisation par chromatographie en phase gazeuse effectuée, on a obtenu une composition comprenant 93,8% en poids d'ester éthylique.

### Exemple 6 : Préparation d'esters de butanediol d'huile de ricin

Cet exemple concerne la préparation du composé (6) de formule suivante :

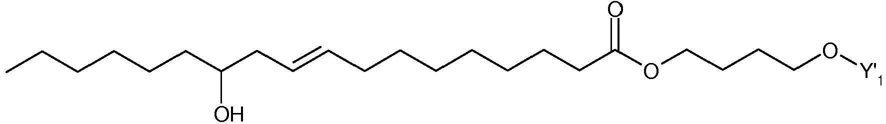

Il s'agit d'un composé de formule (IV') dans laquelle R"₁ représente un groupe alkyle comprenant 8 atomes de carbone et substitué par un groupe OH (sur le carbone 7), A₁ représente un radical alkylène comprenant 7 atomes de carbone, A₂ représente un radical butylène et Y'₁ représente H ou un groupe de formule (A'₁) telle que définie ci-dessus.

### Le produit de départ est le composé (5) tel qu'obtenu dans l'exemple 5.

On a introduit dans un réacteur (500 mL) 300,5 g de composé (5) avec 43,5 g (0,5 mol) de 1,4-butanediol (Aldrich)(composé de formule (III) avec A₂ = butylène). L'ensemble est chauffé à 65°C. On a ensuite ajouté 3,6005 g de MeONa (Aldrich) dans le réacteur et on a alors constaté un changement de couleur du produit (jaune opaque). L'ensemble est ensuite laissé réagir pendant 6 heures à 70-75°C sous agitation (650 tr.min⁻¹) à une pression de 800 à 2 mbar.

On a ensuite effectué une neutralisation avec quelques gouttes de HCI puis un lavage à l'eau pour éliminer les traces de butanediol jusqu'à neutralité. Enfin, l'eau résiduelle a été distillée au rotavapor.

On a ainsi obtenu 260 g d'ester de butanediol d'huile de ricin de formule (6) susmentionnée avec une teneur en eau de 0,30% en poids. Le produit de formule (6) est sous la forme d'un liquide jaune et présente un indice d'acide de 5,05%.

D'après la caractérisation par chromatographie en phase gazeuse effectuée, on a obtenu une composition comprenant 43,9% en poids de diesters (composé (6) avec Y'₁ = (A'₁)), 30,4% en poids de monoesters (composé (6) avec Y'₁ = H) et 25,7% en poids de composé (5).

### Exemple 7 : Préparation d'esters éthyliques d'huile de tournesol époxydés

Cet exemple concerne la préparation du composé (**7**) de formule suivante :

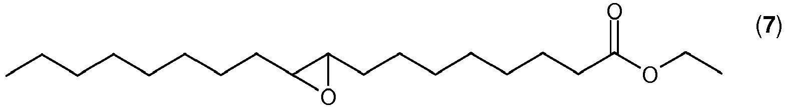

Il s'agit d'un composé de formule (IV"-2) dans laquelle R₁ représente un groupe alkyle comprenant 8 atomes de carbone, A₁ représente un radical alkylène comprenant 7 atomes de carbone et R₂ représente un groupe éthyle.

Le produit de départ est le composé ester éthylique de l'exemple 1bis d'huile de tournesol oléique.

On a introduit dans un réacteur (1 L) 399,6 g du composé (**1**) de l'exemple 1bis (ester éthylique d'huile tournesol oléique - EEHTO) et 20,1 g d'acide formique et laissé réagir l'ensemble à 45°C pendant 1 heure à 500 tr.min⁻¹. On a ensuite additionné par une ampoule à brome au goutte à goutte de l'eau oxygénée pendant 40 minutes (199,2 g de H₂O₂ (BAKER)). L'ensemble a ensuite été laissé réagir pendant 2 heures à 75°C sous agitation à 650 tr.min⁻¹. La réaction étant exothermique, le milieu a été refroidi avec un bain d'eau froide.

On a ensuite effectué un lavage à l'eau jusqu'à neutralité des eaux de lavage. Enfin, l'eau résiduelle a été distillée au rotavapor.

On a ainsi obtenu 410,3 g d'esters éthyliques époxydés d'huile de tournesol oléique de formule (**7**) susmentionnée. Le produit de formule (**7**) est sous la forme d'un liquide orangé et présente un indice d'acide de 0,79%, ainsi qu'une teneur eu eau de 0,41%.

### Exemple 8 : Préparation du diol (8)

Cet exemple concerne la préparation du composé (**8**) de formule suivante :

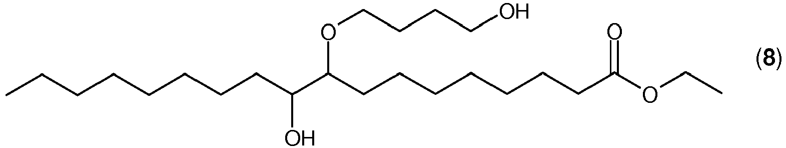

Il s'agit d'un composé de formule (I"-1) dans laquelle R'₁ représente un groupe alkyle comprenant 8 atomes de carbone, A₂ représente un radical butylène, A₁ représente un radical alkylène comprenant 7 atomes de carbone et R₂ représente un groupe éthyle.

Le produit de départ est le composé ester éthylique de l'exemple 7 d'huile de tournesol oléique époxydé.

On a introduit dans un réacteur (2 L) 301,2 g de composé (**7**) avec 12 g (4% en poids) de résine Amberlyst (Aldrich) et 1 201,1 g de 1,4-butanediol distillé (Aldrich). On a laissé l'ensemble réagir à 70°C pendant 4 heures à 500 tr.min⁻¹.

Le butanediol a été distillé à 120-150°C sous 30 mbar et l'ensemble a été lavé à l'eau pour éliminer les traces de butanediol. On a ensuite filtré la résine sur Büchner et, enfin, l'éthanol résiduel a été distillé au rotavapor.

On a ainsi obtenu 270,3 g du polyol de formule (**8**) susmentionnée. Le produit de formule (8) présente un indice d'acide de 0,27% et un indice d'hydroxyle de 255,8 mg KOH/g.

D'après la caractérisation par chromatographie en phase gazeuse effectuée, on a obtenu une composition comprenant moins de 0,1% en poids de butanediol, 22,2% en poids de diesters, 71,7% en poids de monoesters (composé **8**) et 6,1% en poids de composé (**1**).

### Exemple 9 : Préparation d'esters éthyliques d'huile de colza érucique

Cet exemple concerne la préparation du composé (**9**) de formule suivante :

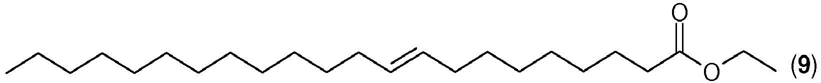

Il s'agit d'un composé de formule (II) dans laquelle R₁ représente un groupe alkyle comprenant 12 atomes de carbone, A₁ représente un radical alkylène comprenant 7 atomes de carbone et R₂ représente un groupe éthyle.

Le produit de départ est l'huile de colza érucique de formule :

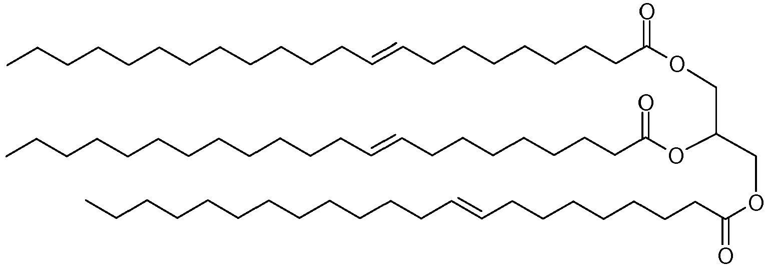

On a introduit dans un réacteur double enveloppe (de 1,5 litre) 1 004,7 g d'huile de colza érucique (HCE)(ITERG, M = 951,8 g.mol⁻¹ - teneur en eau = 0,35% en poids) avec 290,7 g d'éthanol absolu (JT Baker - M = 46,07 g.mol⁻¹). L'ensemble est mélangé sous agitation à 650 tr.min⁻¹ et chauffé à 65°C. On a ensuite ajouté 11,1 g de MeONa (Aldrich - M = 54,02 g.mol⁻¹) dans le réacteur et on a alors constaté un changement de couleur du produit et l'apparition d'un trouble instantané. L'ensemble est ensuite laissé réagir pendant 1 heure à 70°C.

Le mélange réactionnel résultant a ensuite été transvasé dans une ampoule à décanter afin d'éliminer le glycérol et évaporer l'éthanol. On a ensuite effectué une neutralisation avec quelques gouttes de HCI puis un lavage à l'eau jusqu'à neutralité. Enfin, l'eau résiduelle a été distillée au rotavapor.

On a ainsi obtenu 1 026,8 g d'ester éthylique d'huile de colza de formule (9) susmentionnée avec une teneur en eau de 0,27% en poids et un indice d'acide de 1,04%.

D'après la caractérisation par chromatographie en phase gazeuse effectuée, on a obtenu une composition comprenant 98,48% en poids d'ester éthylique.

### Exemple 10 : Préparation d'esters éthyliques d'huile de colza époxydés

Cet exemple concerne la préparation du composé (**10**) de formule suivante :

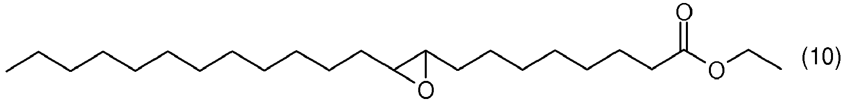

Il s'agit d'un composé de formule (IV"-2) dans laquelle R₁ représente un groupe alkyle comprenant 12 atomes de carbone, A₁ représente un radical alkylène comprenant 7 atomes de carbone et R₂ représente un groupe éthyle.

Le produit de départ est le composé ester éthylique de l'exemple 9 d'huile de colza.

On a introduit dans un réacteur (1 L) 400,5 g de composé (**9**) (ester éthylique d'huile de colza - EECE) et 23,12 g d'acide formique et laissé réagir l'ensemble à 45°C pendant 1 heure à 500 tr.min⁻¹. On a ensuite additionné par une ampoule à brome au goutte à goutte de l'eau oxygénée pendant 40 minutes (211,1 g de H₂O₂ (BAKER)). L'ensemble a ensuite été laissé réagir pendant 3 heures à 75°C sous agitation à 650 tr.min⁻¹. La réaction étant exothermique, le milieu a été refroidi avec un bain d'eau froide.

On a ensuite effectué un lavage à l'eau jusqu'à neutralité des eaux de lavage. Enfin, l'eau résiduelle a été distillée au rotavapor.

On a ainsi obtenu 410,2 g d'esters éthyliques époxydés d'huile de ricin érucique de formule (**10**) susmentionnée. Le produit de formule (**10**) est sous la forme d'un solide blanc à température ambiante et présente un indice d'acide de 1,08%, ainsi qu'une teneur eu eau de 0,22%.

### Exemple 11 : Préparation du diol (11)

Cet exemple concerne la préparation du composé (**11**) de formule suivante :

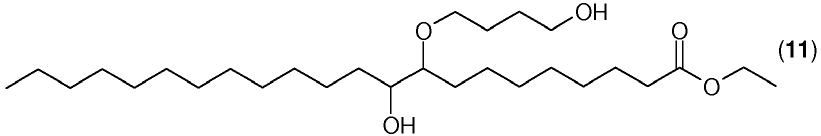

Il s'agit d'un composé de formule (I"-1) dans laquelle R'₁ représente un groupe alkyle comprenant 12 atomes de carbone, A₁ représente un radical butylène, A₁ représente un radical alkylène comprenant 7 atomes de carbone et R₂ représente un groupe éthyle.

Le produit de départ est le composé ester éthylique de l'exemple 10 d'huile de colza érucique époxydé.

On a introduit dans un réacteur (2 L) 350,2 g de composé (**10**) avec 14,2 g (4% en poids) de résine Amberlyst (Aldrich) et 1 484,6 g de 1,4-butanediol distillé (Aldrich). On a laissé l'ensemble réagir à 70°C pendant 4 heures à 500 tr.min⁻¹.

On a alors obtenu deux phases : la phase supérieure contenant le composé 11 et des traces de butanediol et la phase inférieure contenant le butanediol et des traces de composé (**11**).

On a donc distillé la phase supérieure sous agitation magnétique sous vide à 120-140°C sous 30 mbar. On a également distillé la phase inférieure sous vide et sous flux de diazote sans agitation pendant deux jours pour obtenir un produit marron foncé.

L'ensemble a été lavé à l'eau pour éliminer les traces de butanediol. On a ensuite filtré la résine sur Büchner et, enfin, l'éthanol résiduel a été distillé au rotavapor.

On a obtenu via la phase supérieure 126 g d'un liquide jaune clair avec un indice d'acide 0,69% et un indice d'hydroxyle de 190,4 mg KOH/g, ainsi qu'une teneur en eau de 0,64%.
D'après la caractérisation par chromatographie en phase gazeuse effectuée, on a obtenu une composition comprenant moins de 0,1% en poids de butanediol, 6,9% en poids de diesters, 89,1% en poids de monoesters (composé **11**), 0,7% en poids de triglycérides et 3,3% en poids de composé (**9**).

On a obtenu via la phase inférieure 135 g d'un liquide marron foncé avec une teneur en eau de 0,35%.
D'après la caractérisation par chromatographie en phase gazeuse effectuée, on a obtenu une composition comprenant moins de 0,3% en poids de butanediol, 11,4% en poids de diesters, 87,7% en poids de monoesters (composé **11**), 0,4% en poids de triglycérides et 0,3% en poids de composé (**9**).

### Exemple 12 : Préparation de polymères à partir de polyols de formule (I')

En appliquant les mêmes procédures que les exemples 1 à 6 susmentionnés, on a également synthétisé les composés suivants :
Synthèse de polymères issus du polyol

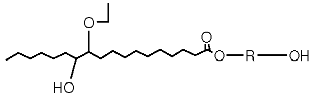

| | | | |
|---|---|---|---|
| Groupement R | C₃H₆ | C₄H₈ | C₆H₁₂ |
| Temps de réaction | 5h | 5h | 5h |
| M_{w} | 20 000 g/mol | 20 000 g/mol | 18 000 g/mol |
| IP | 1,4 | 1,5 | 1,3 |

Synthèse de polymères issus du polyol

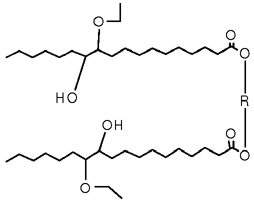

| | | | |
|---|---|---|---|
| Groupement R | C₃H₆ | PEG₃₀₀ | PEG₆₀₀ |
| Temps de réaction | 7h | 8h | 10h |
| M_{w} | 15 000 g/mol | 14 000 g/mol | 14 000 g/mol |
| IP | 1,4 | 1,5 | 1,3 |

Les polyols de l'invention sont utilisés pour préparer des polymères par exemple par réaction avec des isocyanates. Le protocole appliqué est décrit ci-après et peut être appliqué à tout polyol et tout isocyanate.

On a ajouté dans un réacteur de 100 mL le polyol de l'invention et le catalyseur puis l'isocyanate (en particulier l'IPDI) a été additionné dans le réacteur par l'intermédiaire d'un entonnoir. On a maintenu la température du mélange à 60°C par chauffage.

### A partir du polyol monoester avec R= C₃H₆

2 g de monoester ont été introduits dans un réacteur de 100 mL en présence de 2mg de DBTDL (LCPO)(dilaurate de dibutylétain)(0,1% en poids). Puis on a introduit 1,1g d'IPDI (Aldrich - M = 222,29 g.mol⁻¹)(isophorone diisocyanate) (1 équivalent). Le mélange a été placé sous agitation magnétique pendant 5h.

On a effectué un suivi cinétique de la réaction par analyse IR ce qui a permis d'observer la disparition de la bande N=C à 2 269,94 cm⁻¹ et l'apparition de la bande N-H à 3 350 cm⁻¹. Le polymère obtenu a été analysé par chromatographie d'exclusion stérique (les masses molaires obtenues sont répertoriées dans les tableaux ci-dessus)

Le protocole est inchangé pour les cas où R= C₄H₁₀ et R= C₆H₁₂.

### A partir du polyol diester avec R= C₃H₆

2 g de diester ont été introduits dans un réacteur de 100 mL en présence de 2 mg de DBTDL (LCPO)(dilaurate de dibutylétain). Puis on a introduit 586 mg d'IPDI (Aldrich - M = 222,29 g.mol⁻¹) (isophorone diisocyanate) (0,5 équivalent). Le mélange a été placé sous agitation magnétique pendant 7h.

On a effectué un suivi cinétique de la réaction par analyse IR ce qui a permis d'observer la disparition de la bande N=C à 2 269,94 cm⁻¹ et l'apparition de la bande N-H à 3 350 cm⁻¹. Le polymère obtenu a été analysé par chromatographie d'exclusion stérique (les masses molaires obtenues sont répertoriées dans les tableaux ci-dessus)

Le protocole est inchangé pour les cas où R= PEG₃₀₀ et R= PEG₆₀₀.

### Exemple 13 : Préparation de polyuréthanes à partir du diol 8

Les polyols de l'invention sont utilisés pour préparer des polymères par exemple par réaction avec des isocyanates. Le protocole appliqué est décrit ci-après et peut être appliqué à tout polyol et tout isocyanate.

On a ajouté dans un réacteur d'un litre le polyol de l'invention et le catalyseur puis l'isocyanate (en particulier l'IPDI ou le HMDI) a été additionné dans le réacteur par l'intermédiaire d'un entonnoir. L'ensemble a ensuite été agité à 80 t.min⁻¹ sous diazote afin d'homogénéiser le mélange. On a alors observé l'apparition de bulles dans le mélange réactionnel et on a maintenu la température du mélange à 60°C par chauffage.

On a effectué un suivi cinétique de la réaction par analyse IR ce qui a permis d'observer la disparition de la bande N=C à 2 269,94 cm⁻¹ et l'apparition de la bande N-H à 3 350 cm⁻¹.

Plus particulièrement, ce protocole a été appliqué en utilisant comme polyol le diol 8 de l'exemple 8 et en faisant varier la nature de l'isocyanate (IPDI et HMDI), ainsi que le temps de réaction et le ratio OH:NCO.

Le catalyseur utilisé est le DBTDL (LCPO)(dilaurate de dibutylétain) à 0,1% en poids.

Les résultats obtenus sont résumés ci-après dans les tableaux 1 et 2.

Le tableau 1 correspond à la synthèse de polyuréthane par réaction avec l'IPDI (Aldrich - M = 222,29 g.mol⁻¹)(isophorone diisocyanate) :

| N° | OH:NC O | Durée de réaction (h) | Solubilit é (DCM, THF, DMF) | Analyse IR | Analyse CPG (Mw) | Viscosité (cst) | | | |
|---|---|---|---|---|---|---|---|---|---|
| | | | | | | 80°C | | 100°C | |
| | | | | | | Cisaillement (s⁻¹) | | | |
| | | | | | | 1 | 10 | 1 | 10 |
| 1 | 1:0,2 | 7 | soluble | Pas de bande isocyanate | 1 830 | - | 9,5 | - | 4,5 |
| 2 | 1:0,3 | 7 | soluble | Pas de bande isocyanate | 2 300 | - | 23,5 | - | 11,5 |
| 3 | 1:0,5 | 7 | soluble | Pas de bande isocyanate | 5 240 | - | 160 | - | 70 |
| 4 | 1:0,65 | 7 | soluble | Pas de bande isocyanate | 10 820 | 1050 | 775 | - | 280 |
| 5 | 1:0,69 | 12 | soluble | Pas de bande isocyanate | 12 570 | - | - | - | - |

| | | | | | | | | | |
|---|---|---|---|---|---|---|---|---|---|
| DCM : dichlorométhane THF : tétrahydrofurane DMF : diméthylformamide | | | | | | | | | |

Les viscosités appropriées sont obtenues pour un ratio OH:NCO inférieur à 1:0,70.

Le tableau 2 correspond à la synthèse de polyuréthane par réaction avec l'HMDI (hexaméthylène diisocyanate) :

| N° | OH:NC O | Durée de réaction (h) | Solubilit é (DCM, THF, DMF) | Analyse IR | Analyse CPG (Mw) | Viscosité (cst) | | | |
|---|---|---|---|---|---|---|---|---|---|
| | | | | | | 80°C | | 100°C | |
| | | | | | | Cisaillement (s⁻¹) | | | |
| | | | | | | 1 | 10 | 1 | 10 |
| 1 | 1:0,3 | 6 | soluble | Pas de bande isocyanate | 2 740 | - | 31,7 | - | 14,5 |
| 2 | 1:0,5 | 6 | soluble | Pas de bande isocyanate | 7 200 | 287 | 385 | 93 | 188 |
| 3 | 1:0,65 | 9 | soluble | Pas de bande isocyanate | 13 550 | 3 474 | 3 055 | 879 | 891 |

### Exemple 14 : Préparation d'un diol de formule (I-2) avec deux fonctions alcools secondaires

Le protocole décrit ci-après a été utilisé pour synthétiser des composés de formule (I-2), A₁ représentant un radical C₇H₁₄ et R₁ représentant un groupe alkyle comprenant 9 atomes de carbone.

Dans la formule (I-2), A₂ peut représenter un radical choisi parmi les radicaux suivants : C₃H₆, C₄H₈, C₅H₁₀, C₆H₁₂, H₂C-(CH₂OCH₂)₆-CH₂, H₂C-(CH₂OCH₂)₁₃-CH₂, H₂C-(CH₂OCH₂)₄₅-CH₂ ou H₂C-CₑH₄-CH₂.

### Etape de transestérification :

Les diesters sont issus de la transestérification d'un ester méthylique oléique et d'un diol (propanediol, butanediol, pentanediol, hexanediol, polyoxyde d'éthylène (300g/mol, 600g/mol et 2000 g/mol)). La synthèse a fait intervenir 0,1 mol d'ester méthylique oléique et 0,05 mol de diol, en présence d'oxyde de magnésium MgO (catalyseur, 1% en masse par rapport à la masse d'ester méthylique). Le milieu a été gardé sous agitation à 160°C, sous flux d'azote, pendant 7 heures. Le méthanol formé par la réaction a été enlevé du milieu réactionnel grâce à une trappe Dean Stark. La formation du diester a été suivie par RMN ¹H. Au bout de 7h, le milieu a été placé à 200°C sous vide dynamique pendant 1h afin d'éliminer l'ester méthylique oléique et les diols résiduels. Le catalyseur a été éliminé par filtration.

Pour la synthèse du diester à partir d'ester méthylique et de 1,4-benzènediméthanol (A₂= H₂C-C₆H₄-CH₂), la température du milieu lors de la réaction était de 140°C afin de ne pas sublimer le 1,4-benzènediméthanol.

### Etape d'époxydation :

10 mmol de diester synthétisé précédemment ont été mélangés avec 3 mmol d'acide formique (HCOOH). Le milieu a été chauffé à 40°C pendant 1h. Puis on a ajouté goutte à goutte 10 mmol de peroxyde d'hydrogène (H₂O₂). La température a été élevée à 70°C pendant 2h. La formation de l'époxyde a été suivie par RMN ¹H. Lorsque la réaction est terminée, on a procédé à un lavage eau-dichlorométhane afin d'éliminer le peracide.

### Etape d'hydroxylation :

Pour l'étape d'ouverture de l'époxyde, 10 mmol de diesters époxydés ont été dissous dans 100 mmol d'éthanol, en présence de résine échangeuse d'ion (Amberlyst 15 Dry, 4% en masse par rapport à la masse de diesters). Le milieu réactionnel a été placé sous agitation, à 75°C, pendant 20 heures. L'ouverture de l'époxyde a été suivie par RMN ¹H. Quand la réaction a été terminée, le catalyseur a été retiré par filtration. L'excès d'éthanol a ensuite été évaporé sous pression réduite. Les diesters hydroxylés ont alors été analysés par RMN ¹H et par chromatographie d'exclusion stérique. Leur indice hydroxyle a été déterminé.

### Exemple 15 : Préparation d'un diol de formule (I-1) avec deux fonctions alcools secondaires

Le protocole décrit ci-après a été utilisé pour synthétiser des composés de formule (I-1), A₁ représentant un radical C₇H₁₄ et R₁ représentant un groupe alkyle comprenant 9 atomes de carbone.

Dans la formule (I-1), A₂ peut représenter un radical choisi parmi les radicaux suivants : C₃H₆, C₄H₈, C₅H₁₀, C₆H₁₂, H₂C-(CH₂OCH₂)₆-CH₂, H₂C-(CH₂OCH₂)₁₃-CH₂, H₂C-(CH₂OCH₂)₄₅-CH₂ ou H₂C-C₆H₄-CH₂.

Les protocoles de synthèse sont les mêmes que ceux indiqués pour l'exemple 14, exceptés pour l'étape de transestérification. La synthèse a fait intervenir 0,1 mol d'ester méthylique oléique et 1,5 mol de diol, afin de favoriser la formation de monoesters par rapport aux diesters.

### Exemple 16 : Préparation de composés bisépoxydes de formule (V-3)

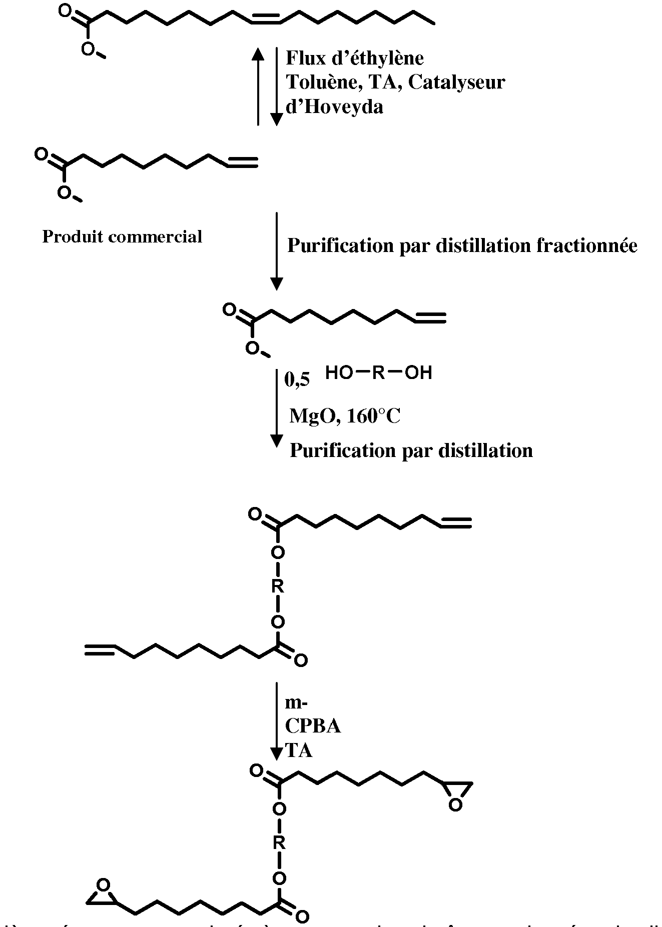

La première étape a consisté à couper la chaîne carbonée de l'ester méthylique oléique au niveau de la double liaison interne afin d'obtenir une double liaison terminale. Une réaction de métathèse entre l'éthylène et la double liaison interne de l'ester méthylique oléique en présence de catalyseur d'Hoveyda a conduit à la formation de décène et de 10-undécénoate de méthyle. Cette réaction s'est déroulée sous agitation à température ambiante. A l'état d'équilibre, le milieu est composé de 48% d'ester méthylique oléique de départ, 26% de décène et 26% de 10-undécénoate de méthyle. Ce dernier a été extrait par distillation sous vide : la première fraction à 100°C contenait du décène ; le 10-undecenoate de méthyle a été récupéré lorsque la température a atteint 180°C. Le résidu était composé d'ester méthylique oléique.

On a ensuite procédé à une réaction de transestérification du 10-undécénoate de méthyle avec un diol (aliphatique, aromatique, phénol d'origine naturelle, etc). La réaction s'est déroulée sous vide en présence de 0,5 équivalent de diol afin de favoriser la formation de diesters ; elle a été catalysée par 1_{wt}% d'oxyde de magnésium. La température du milieu a été élevée à 160°C, le méthanol produit a été enlevé en continu à l'aide d'une trappe Dean Stark. Au bout de 7h, la température a été élevée à 180°C afin d'éliminer le 10-undécénoate de méthyle résiduel.

La dernière étape a consisté en l'époxydation des doubles liaisons du produit obtenu par transestérification. Elle s'est effectué en présence d'acide métachloroperbenzoïque (m-CPBA) (1,2eq par double liaison), dans du dichlorométhane, à température ambiante. La conversion des doubles liaisons était totale au bout de 3h. L'excès de m-CPBA a été réduit en acide carboxylique correspondant avec une solution saturée de sulfate de sodium. La phase organique a été extraite avec du dichlorométhane puis l'acide carboxylique résiduel a été transformé en chlorobenzoate de sodium (soluble dans l'eau) grâce à deux lavages avec une solution saturée de bicarbonate de soude.

### Exemple 17 : Préparation d'esters éthyliques d'huile de tournesol oléique

Cet exemple concerne la préparation du composé (1) de l'exemple 1 susmentionné à partir d'huile de tournesol oléique (HTO).

On a introduit dans un réacteur double enveloppe 1 001,2 g d'huile de tournesol oléique (HTO)(ITERG, M = 884,82 g.mol⁻¹ - teneur en eau = 0,35% en poids) avec 314,0 g d'éthanol absolu (JT Baker - M = 46,07 g.mol⁻¹). L'ensemble est mélangé sous agitation à 650 tr.min⁻¹ et chauffé à 65°C. On a ensuite ajouté 11,5 g de MeONa (Aldrich - M = 54,02 g.mol⁻¹) dans le réacteur et on a alors constaté un changement de couleur du produit et l'apparition d'un trouble instantané. L'ensemble a ensuite été laissé réagir pendant 1 heure à 70°C.

Le mélange réactionnel résultant a ensuite été transvasé dans une ampoule à décanter afin d'éliminer le glycérol et évaporer l'éthanol. On a ensuite effectué une neutralisation avec quelques gouttes de HCI puis un lavage à l'eau jusqu'à neutralité. Enfin, l'eau résiduelle a été distillée au rotavapor.

On a ainsi obtenu 1 056,5 g d'ester éthylique d'huile de tournesol de formule (1) susmentionnée avec une teneur en eau de 0,15% en poids.

D'après la caractérisation par chromatographie en phase gazeuse effectuée, on a obtenu une composition comprenant 97,8% en poids d'ester éthylique.

### Exemple 18 : Préparation d'esters éthyliques d'huile de tournesol époxydés

Cet exemple concerne la préparation du composé (7) de l'exemple 7 à partir du composé (1) de l'exemple 18.

On a introduit dans un réacteur (2 L) 800,5 g du composé (7) de l'exemple 7 (ester éthylique d'huile tournesol oléique - EEHTO) et 39,4 g d'acide formique et laissé réagir l'ensemble à 45°C pendant 1 heure à 500 tr.min⁻¹. On a ensuite additionné par une ampoule à brome au goutte à goutte de l'eau oxygénée pendant 36 minutes (355,5 g de H₂O₂ (BAKER)). L'ensemble a ensuite été laissé réagir pendant 2 heures à 75°C sous agitation à 650 tr.min⁻¹. La réaction étant exothermique, le milieu a été refroidi avec un bain d'eau froide.

On a ensuite effectué un lavage à l'eau jusqu'à neutralité des eaux de lavage. Enfin, l'eau résiduelle a été distillée au rotavapor.

On a ainsi obtenu 1 056,5 g d'esters éthyliques époxydés d'huile de tournesol oléique de formule (7) susmentionnée. Le produit de formule (7) est sous la forme d'un liquide jaune clair et présente une teneur eu eau de 0,11%.

### Exemple 19 : Préparation du diol (8)

Cet exemple concerne la préparation du diol (8) de l'exemple 8 à partir du composé (7) de l'exemple 18.

On a introduit dans un réacteur (2 L) 100,4 g de composé (7) avec 4,1 g (4% en poids) de résine Amberlyst (Aldrich) et 416,1 g de 1,4-butanediol distillé (Aldrich). On a laissé l'ensemble réagir à 70°C pendant 4 heures à 650 tr.min⁻¹.

L'ensemble a été lavé à l'eau pour éliminer les traces de butanediol, et, enfin, l'éthanol résiduel a été distillé au rotavapor.

On a ainsi obtenu 93,0 g du polyol de formule (8) susmentionnée.

D'après la caractérisation par chromatographie CLHP effectuée, on a obtenu une composition comprenant 1,1% en poids de butanediol, 6,8% en poids de diesters, 70,5% en poids de monoesters (composé 8) et 2,0% en poids de composé (1).

### Exemple 20 : Préparation d'esters éthyliques d'huile de colza époxydés

Cet exemple concerne la préparation du composé (10) de l'exemple 10 à partir d'esters éthyliques d'huile de colza érucique.

On a introduit dans un réacteur (1 L) 400,6 g de composé (9) (ester éthylique d'huile de colza - EECE) et 27,0 g d'acide formique et laissé réagir l'ensemble à 45°C pendant 1 heure à 500 tr.min⁻¹. On a ensuite additionné par une ampoule à brome au goutte à goutte de l'eau oxygénée pendant 45 minutes (211,1 g de H₂O₂ (BAKER)). L'ensemble a ensuite été laissé réagir pendant 3 heures à 75°C sous agitation à 650 tr.min⁻¹. La réaction étant exothermique, le milieu a été refroidi avec un bain d'eau froide.

On a ensuite effectué un lavage à l'eau jusqu'à neutralité des eaux de lavage. Enfin, l'eau résiduelle a été distillée au rotavapor.

On a ainsi obtenu 391,1 g d'esters éthyliques époxydés d'huile de ricin érucique de formule (10) (cf. exemple 10).

### Exemple 21 : Préparation du diol (11)

Cet exemple concerne la préparation du composé (11) de l'exemple 11 à partir de l'ester éthylique de l'exemple 10 d'huile de colza érucique époxydé.

On a introduit dans un réacteur (2 L) 350,2 g de composé (10) avec 14,3 g (4% en poids) de résine Amberlyst (Aldrich) et 514,5 g de 1,4-butanediol distillé (Aldrich). On a laissé l'ensemble réagir à 70°C pendant 4 heures à 650 tr.min⁻¹.

L'ensemble a été versé dans une ampoule à décanter, dans laquelle on a ajouté 200 mL d'eau tiède puis 100 mL de butanol. Après agitation et déphasage au repos, on a observé deux phases. La phase supérieure a été mise de côté et la phase inférieure a été lavée avec 2 x 50 mL de butanol. Les phases supérieures ont ensuite été regroupées, lavées à l'eau tiède afin d'éliminer les traces de butanediol, puis séchées au rotavapor.

On a ainsi obtenu 272,1 g d'un liquide jaune clair avec un indice d'hydroxyle de 187,9 mg KOH/g, ainsi qu'une teneur en eau de 0,66%.
D'après la caractérisation par chromatographie en phase gazeuse effectuée, on a obtenu une composition comprenant moins de 0,1% en poids de butanediol, 9,2% en poids de diesters et 66,1% en poids de monoesters (composé 11).

### Exemple 22 : Synthèse de polyuréthanes à partir des diols des exemples 19 et 21

### Protocole de synthèse

On a chargé dans un flacon tricol (250 mL) avec un agitateur mécanique et une entrée d'azote du dilaurate de dibutylétain (0,003 g, 0,1% en masse par rapport aux monomères), le polyol (de l'exemple 19 ou 21)(18,0 g, indice hydroxyle = 215,9) et de l'isophorone diisocyanate (IPDI)(6,54 g, OH/NCO=1:0,85). Le rapport OH/NCO a été calculé sur la base de l'indice d'hydroxyle du polyol. Le mélange réactionnel a été agité à température ambiante sous flux d'azote et chauffé à 60°C pendant 9 heures. La polymérisation a été contrôlée par spectroscopie IR sur la base de la bande isocyanate. Après achèvement de la réaction, on a ajouté 0,7 g d'octyl dodécanol pour stopper la réaction avec un chauffage supplémentaire pendant 12 heures additionnelles. Le polymère obtenu a été caractérisé par IR, RMN et CPG.

Le même protocole a été appliqué en utilisant le HDMI comme diisocyanate à la place de l'IPDI.

Le tableau 3 ci-après illustre les résultats obtenus pour la synthèse de polyuréthane par réaction avec l'IPDI ou l'HDMI comme isocyanate et les polyols des exemples 19 et 21 :

| Polyol | Ratio OH/NCO | diisocyanate | Analyse CPG | | Viscosité (cst) | | | | | |
|---|---|---|---|---|---|---|---|---|---|---|
| | | | Mw | Mw/Mn | | | | | | |
| | | | | | 30°C | | 80°C | | 100°C | |
| | | | | | Cisaillement (1/s) | | | | | |
| | | | | | 1 | 10 | 1 | 10 | 1 | 10 |
| Ex,19 | 1:0,85 | IPDI | 3280 | 1,61 | 5850* | - | 105 | 93 | - | 31,5 |
| Ex,19 | 1:0,75 | HMDI | 3210 | 1,35 | 6550* | - | 98 | 92 | 38 | 33 |
| Ex,19 | 1:0,85 | HMDI | 4160 | 1,47 | - | - | 250 | 220 | 95 | 60 |
| Ex,21 | 1:0,7 | IPDI | 3730 | 1,32 | - | - | - | - | - | - |

## Revendications

1. Procédé de préparation d'un polyol répondant à la formule générale (I) suivante : 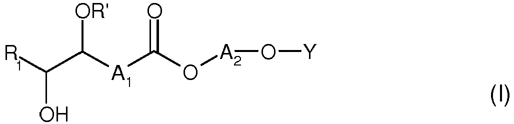 dans laquelle :
- R₁ représente H ou un groupe alkyle, linéaire ou ramifié, comprenant de 2 à 14 atomes de carbone,
- R' représente un groupe alkyle, linéaire ou ramifié, comprenant de 1 à 18 atomes de carbone,
- A₁ représente un radical alkylène divalent linéaire ou ramifié, comprenant de 2 à 14 atomes de carbone,
- A₂ représente un radical alkylène divalent linéaire ou ramifié, comprenant de 1 à 10 atomes de carbone, le cas échéant comprenant un ou plusieurs substituants choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45,
- Y représente un atome d'hydrogène ou un groupe de formule (A) 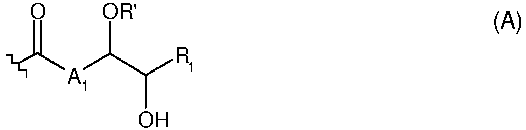
A₁, R' et R₁ étant tels que définis ci-dessus,
ledit procédé comprenant les étapes suivantes :
a) une étape de transestérification d'un composé de formule (II) suivante : 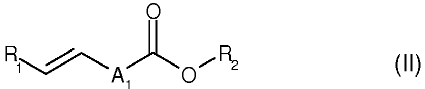
R₁ et A₁ étant tels que définis ci-dessus, et R₂ représente un groupe alkyle, linéaire ou ramifié, comprenant de 1 à 10, de référence de 1 à 6, atomes de carbone,
avec un diol de formule (III) suivante :
HO-A₂-OH (III)
pour obtenir un composé de formule (IV) suivante : 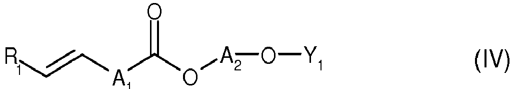
A₁, A₂ et R₁ étant tels que définis ci-dessus dans la formule (I),
Y₁ représentant un atome d'hydrogène ou un groupe de formule (A₁) 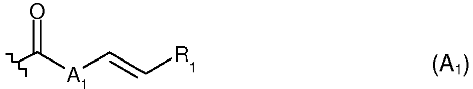
A₁ et R₁ étant tel que défini ci-dessus,
b) une étape d'époxydation du composé de formule (IV) susmentionnée pour obtenir un composé de formule (V) suivante : 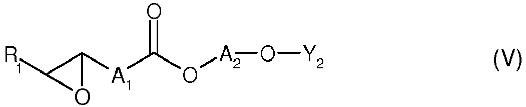
A₁, A₂ et R₁ étant tels que définis ci-dessus,
Y₂ représentant un atome d'hydrogène ou un groupe de formule (A₂) 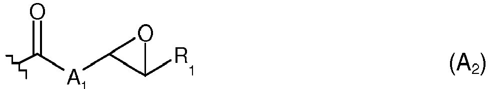
A₁ et R₁ étant tels que définis ci-dessus,
c) une étape d'ouverture du cycle époxyde avec un alcool de formule R'OH, R' étant tel que défini ci-dessus, pour obtenir un composé de formule (I) telle que définie ci-dessus, et
d) une étape de récupération du composé de formule (I) telle que définie ci-dessus,

2. Procédé de préparation d'un diol selon la revendication 1, **caractérisé en ce que** le diol répond à la formule (I-1) suivante : 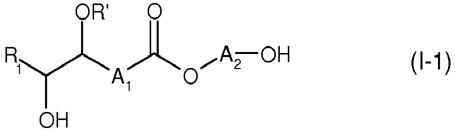 A₁, A₂, R₁ et R' étant tels que définis dans la revendication 1,
ou à la formule (I-2) suivante : 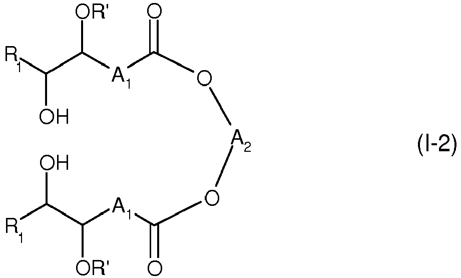 A₁, A₂, R₁ et R' étant tels que définis dans la revendication 1,

3. Procédé de préparation d'un polyol selon l'une quelconque des revendications 1 ou 2, dans lequel l'étape a) est effectuée en présence d'un catalyseur choisi dans le groupe constitué de l'oxyde de magnésium, de l'acétate de zinc et du méthanolate de sodium, et de préférence en ce que l'étape a) est effectuée à une température comprise de 150 à 200°C sous flux d'azote, et plus particulièrement sans solvant,

4. Procédé de préparation d'un polyol selon l'une quelconque des revendications 1 à 3, dans lequel l'étape b) est effectuée en présence d'un peracide, notamment choisi dans le groupe constitué de l'acide métachloroperbenzoïque (m-CPBA) et du peracide magnésium monoperoxyphthalate hexahydrate (MMPP),

5. Procédé de préparation d'un polyol selon l'une quelconque des revendications 1 à 4, dans lequel l'étape c) est effectuée en présence d'un catalyseur choisi dans le groupe constitué d'un catalyseur acide de type résine échangeuse d'ions protonique, notamment avec des fonctions acide sulfonique, des catalyseurs hétérogènes, de l'acide para-tolènesulfonique (APTS) et de l'acide méthanesulfonique (AMS), à une température comprise de 20°C à 120°C, et de préférence à 70°C,

6. Composé répondant à la formule générale (I) suivante : dans laquelle : 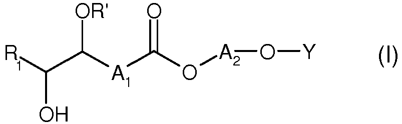
- R₁ représente un groupe alkyle, linéaire ou ramifié, comprenant de 2 à 14 atomes de carbone,
- R' représente un groupe alkyle, linéaire ou ramifié, comprenant de 1 à 18 atomes de carbone,
- A₁ représente un radical alkylène divalent linéaire ou ramifié, comprenant de 2 à 14 atomes de carbone,
- A₂ représente un radical alkylène divalent linéaire ou ramifié, comprenant de 1 à 10 atomes de carbone, le cas échéant comprenant un ou plusieurs substituants choisis dans le groupe constitué du radical phénylène et du radical de formule -(CH₂OCH₂)ₙ-, n représentant un nombre entier compris de 1 à 100, de préférence de 6 à 50, et préférentiellement égal à 6, 13 ou 45, et
- Y représente un atome d'hydrogène ou un groupe de formule (A) 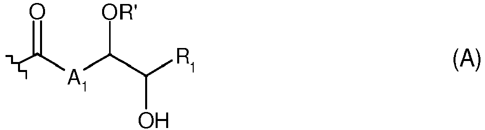 A₁, R' et R₁ étant tels que définis ci-dessus,

7. Composés intermédiaires répondant à la formule suivante : 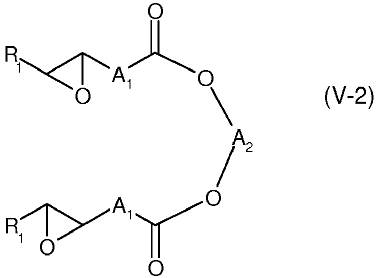 R₁, A₁ et A₂ étant tels que définis dans la revendication 1,

8. Polymères tels qu'obtenus par polymérisation d'un composé tel que défini dans la revendication 6 et d'un (poly)isocyanate,

## Patentansprüche

1. Verfahren zur Herstellung eines Polyols, das der folgenden allgemeinen Formel (I) entspricht: 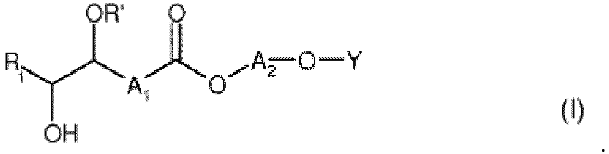 In der:
- R₁ H oder eine lineare oder verzweigte Alkylgruppe, die 2 bis 14 Kohlenstoffatome umfasst, repräsentiert,
- R' eine lineare oder verzweigte Alkylgruppe, die 1 bis 18 Kohlenstoffatome umfasst, repräsentiert,
- A₁ ein bivalentes lineares oder verzweigtes Alkylenradikal, das 2 bis 14 Kohlenstoffatome umfasst, repräsentiert,
- A₂ ein bivalentes lineares oder verzweigtes Alkylenradikal, umfassend 1 bis 10 Kohlenstoffatome, repräsentiert, wobei gegebenenfalls ein oder mehrere Substituenten umfasst sind, die aus der Gruppe bestehend aus Vinylen und dem Radikal der Formel -(CH₂OCH₂)_{N}- ausgewählt sind, wobei N eine ganze Zahl zwischen 1 und 100, vorzugsweise zwischen 6 bis 50 und noch bevorzugter gleich 6, 13 oder 45 darstellt,
- Y ein Wasserstoffatom oder eine Gruppe der Formel (A) 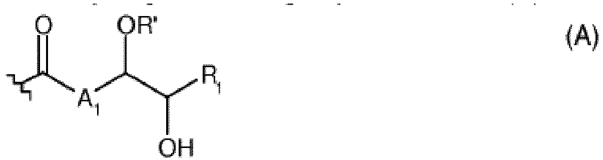 darstellt,
wobei A₁, R' und R₁ derart sind, wie oben definiert,
wobei das Verfahren die folgenden Schritte umfasst:
a) einen Schritt der Umesterung einer Verbindung der folgenden Formel (II) 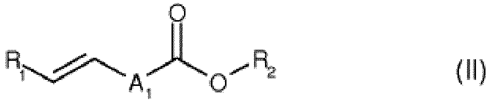
wobei R₁ und A₁ derart definiert sind wie oben und R₂ eine lineare oder verzweigte Alkylgruppe mit 1 bis 10, vorzugsweise 1 bis 6 Kohlenstoffatomen,
mit einem Diol der folgenden Formel (III)
HO-A₂-O_{H} (III)
um eine Verbindung der folgenden Formel (IV) zu erhalten 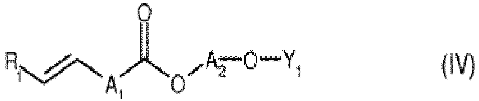
wobei A₁, A₂ und R₁ derart sind, wie oben in der Formel (I) definiert ist,
Y₁ ein Wasserstoffatom oder eine Gruppe der Formel (A₁) darstellt 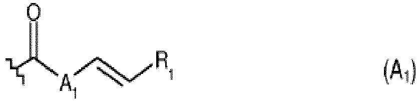
wobei A₁ und R₁ so sind, wie oben definiert,
b) einen Schritt der Epoxidation der Verbindung der oben erwähnten Formel (IV), um eine Verbindung der folgenden Formel (V) zu erhalten: 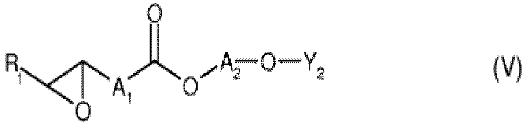
wobei A₁, A₂ und R₁ so sind, wie oben definiert,
wobei Y₂ ein Wasserstoffatom oder eine Gruppe der Formel (A₂) darstellt: 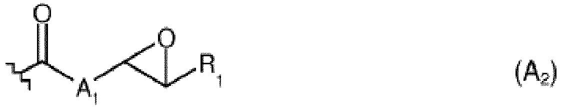
wobei A₁ und R₁ so sind, wie oben definiert,
c) einen Schritt der Öffnung des Epoxidzyklus mit einem Alkohol der Formel R'OH, wobei R' so ist, wie oben definiert, um eine Verbindung der Formel (I) zu erhalten, wie sie oben definiert ist, und
d) einen Schritt der Gewinnung der Verbindung der Formel (I), wie oben definiert.

2. Verfahren zur Herstellung eines Diols nach Anspruch 1, **dadurch gekennzeichnet, dass** das Diol der folgenden Formel (I-1) entspricht, 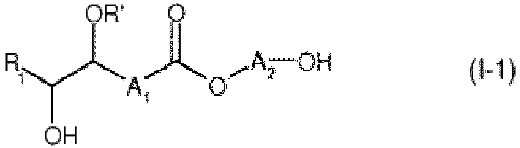 wobei A₁ A₂, R₁ und R' so sind, wie in Anspruch 1 definiert,
oder der folgenden Formel (I-2) 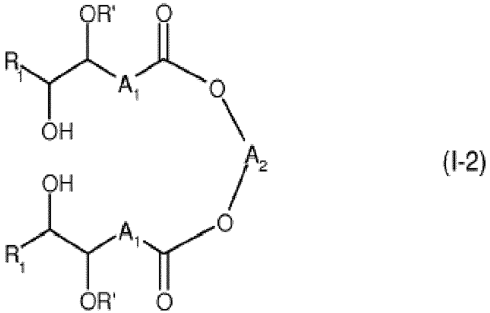 wobei A₁, A₂, R₁ und R' so sind, wie im Anspruch 1 definiert.

3. Verfahren zur Herstellung eines Polyols nach einem beliebigen der Ansprüche 1 oder 2, bei dem der Schritt a) in Anwesenheit eines Katalysators durchgeführt wird, der aus der Gruppe ausgewählt ist, die aus Magnesiumoxid, Zinkacetat und Natrium-Methanolat ausgewählt ist, und vorzugsweise dass der Schritt a) bei einer Temperatur zwischen 150°C und 200°C im Stickstoffstrom und insbesondere ohne Lösungsmittel durchgeführt wird.

4. Verfahren zur Herstellung eines Polyols nach einem beliebigen der Ansprüche 1 bis 3, bei dem der Schritt b) in Anwesenheit einer Persäure, insbesondere ausgewählt aus der Gruppe, bestehend aus Meta-Chlorperbenzolsäure (M-CPBA) und Persäuremagnesium-Monoperoxyphthalate-Hexahydrate (MMPP).

5. Verfahren zur Herstellung eines Polyols nach einem beliebigen der Ansprüche 1 bis 4, bei dem Schritt c) in Anwesenheit eines Katalysators, der aus der Gruppe bestehend aus einem Säurekatalysator der Art eines Protonenaustauscherharzes, insbesondere mit Sulfonsäurefunktionen, heterogenen Katalysatoren, Paratolensulfonsäure (APTS) und Methansulfonsäure (AMS) ausgewählt ist, bei einer Temperatur zwischen 20°C und 120°C und vorzugsweise bei 70°C durchgeführt wird.

6. Verbindung, die der folgenden allgemeinen Formel (I) entspricht. 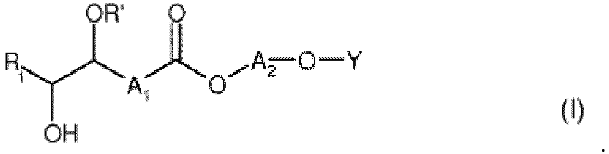 In der:
- R₁H oder eine lineare oder verzweigte Alkylgruppe, die 2 bis 14 Kohlenstoffatome umfasst, repräsentiert,
- R' eine lineare oder verzweigte Alkylgruppe, die 1 bis 18 Kohlenstoffatome umfasst, repräsentiert,
- A₁ ein bivalentes lineares oder verzweigtes Alkylen, das 2 bis 14 Kohlenstoffatome umfasst, repräsentiert,
- A₂ ein bivalentes lineares oder verzweigtes Alkylen, und das 1 bis 10 Kohlenstoffatome umfasst, repräsentiert, wobei gegebenenfalls ein oder mehrere Substituenten umfasst sind, die aus der Gruppe bestehend aus Vinylen und dem Radikal der Formel - (CH₂OCH₂)_{N} - ausgewählt sind, wobei N eine ganze Zahl zwischen 1 und 100, vorzugsweise zwischen 6 bis 50 und noch bevorzugter gleich 6, 13 oder 45 darstellt,
- Y ein Wasserstoffatom oder eine Gruppe der Formel (A) 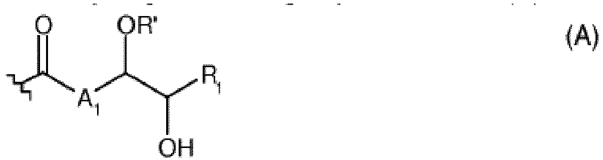 darstellt,
wobei A₁, R' und R₁ derart sind, wie oben definiert,

7. Zwischenprodukte, die der folgenden Formel entsprechen: 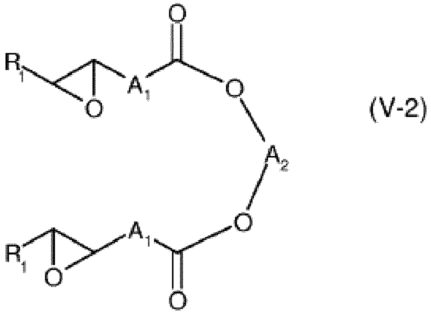 wobei R₁, A₁ und A₂ so sind, wie sie in Anspruch 1 definiert sind.

8. Polymere, wie sie durch Polymerisation einer Verbindung, wie sie in Anspruch 6 definiert ist, und eines (Poly)Isocyanats erhalten werden.

## Claims

1. Method for preparation of a polyol corresponding to the following general formula (I): 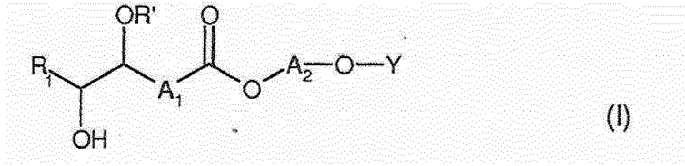 in which:
- R₁ represents H or an alkyl group, linear or branched, comprising 2 to 14 carbon atoms,
- R' represents an alkyl group, linear or branched, comprising 1 to 18 carbon atoms,
- A₁ represents a bivalent alkylene radical, linear or branched, comprising 2 to 14 carbon atoms,
- A₂ represents a bivalent alkylene radical, linear or branched, comprising 1 to 10 carbon atoms, if necessary comprising one or more substituents chosen from the group consisting of the phenylene radical and of the radical of the formula
-(CH₂OCH₂)ₙ-, n representing a whole number between 1 to 100, preferably 6 to 50, and preferably equal to 6, 13 or 45,
- Y represents a hydrogen atom or a group of formula (A) 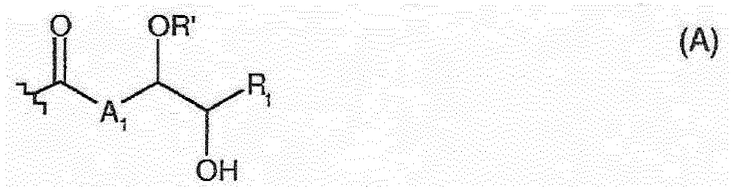
A₁, R' and R₁ being as defined above,
said method comprising the following steps:
a) a transesterification step of a compound of the following formula (II): 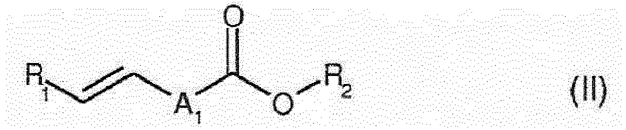
R₁ and A₁ being as defined above, and R₂ represents an alkyl group, linear or branched, comprising 1 to 10, preferably 1 to 6, carbon atoms,
with a diol of the following formula (III):
HO-A₂-OH (III)
in order to obtain a compound of the following formula (IV): 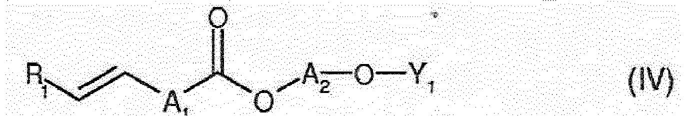
A₁, A₂ and R₁ being as defined above in the formula (I),
Y₁ representing a hydrogen atom or a group of formula (A₁) 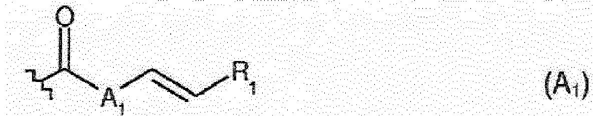
A₁ and R₁ being as defined above,
b) an epoxidation step of the compound of the above-mentioned formula (IV) in order to obtain a compound of the following formula (V): 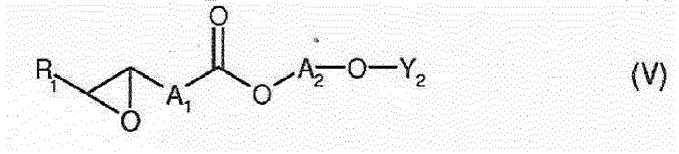
A₁, A₂ and R₁ being as defined above,
Y₂ representing a hydrogen atom or a group of formula (A₂) 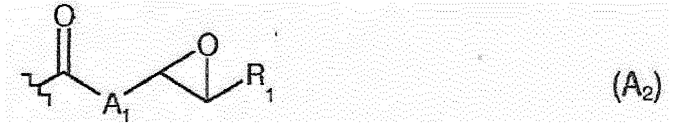
A₁ and R₁ being as defined above,
c) a step of opening the epoxide ring with an alcohol of formula R'OH, R' being as defined above, in order to obtain a compound of formula (I) as defined above, and
d) a step of recovery of the compound of formula (I) as defined above.

2. Method for preparation of a diol according to claim 1, **characterised in that** the diol corresponds to the following formula (I-1): 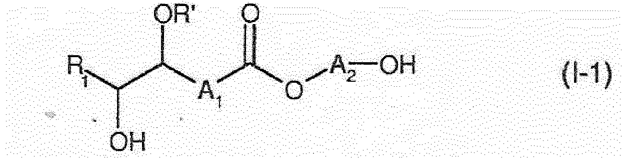 A₁, A₂, R₁ and R' being as defined in claim 1,
or to the following formula (I-2): 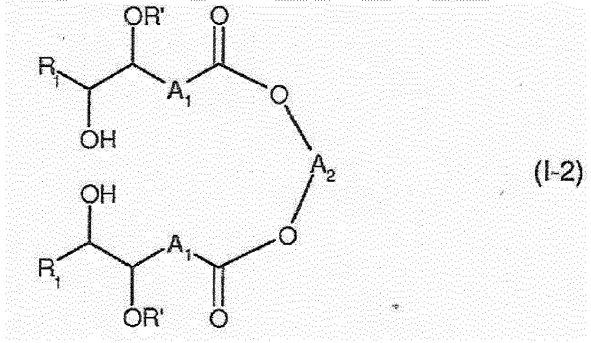 A₁, A₂, R₁ and R' being as defined in claim 1.

3. Method for preparation of a polyol according to any of the claims 1 or 2, in which step a) is implemented in the presence of a catalyst chosen from the group consisting of magnesium oxide, zinc acetate and sodium methanolate, and preferably in that step a) is implemented at a temperature between 150 to 200°C under nitrogen flow, and more particularly without solvent.

4. Method for preparation of a polyol according to any of the claims 1 to 3, in which step b) is implemented in the presence of a peracid, in particular chosen from the group consisting of metachloroperbenzoic acid (m-CPBA) and magnesium monoperoxyphthalate hexahydrate peracid (MMPP).

5. Method for preparation of a polyol according to any of the claims 1 to 4, in which step c) is implemented in the presence of a catalyst chosen from the group consisting of an acid catalyst of the protonic ion exchange resin type, in particular with sulphonic acid functions, heterogeneous catalysts, para-toluenesulphonic acid (APTS) and methanesulphonic acid (AMS), at a temperature between 20°C to 120°C, and preferably at 70°C.

6. Compound corresponding to the following general formula (I): 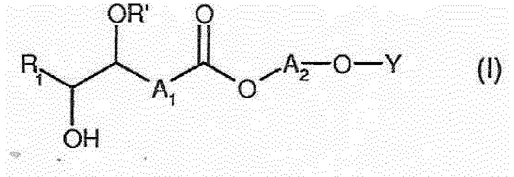 in which
- R₁ represents an alkyl group, linear or branched, comprising 2 to 14 carbon atoms,
- R' represents an alkyl group, linear or branched, comprising 1 to 18 carbon atoms,
- A₁ represents a bivalent alkylene radical, linear or branched, comprising 2 to 14 carbon atoms,
- A₂ represents a bivalent alkylene radical, linear or branched, comprising 1 to 10 carbon atoms, if necessary comprising one or more substituents chosen from the group consisting of the phenylene radical and of the radical of the formula
-(CH₂OCH₂)ₙ-, n representing a whole number between 1 to 100, preferably 6 to 50, and preferably equal to 6, 13 or 45, and
- Y represents a hydrogen atom or a group of formula (A) 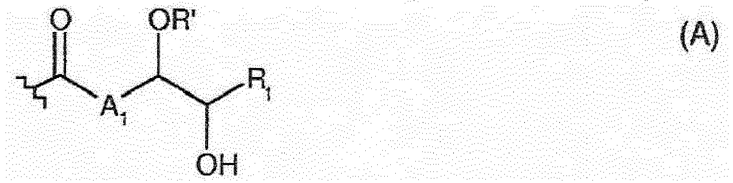 A₁, R' and R₁ being as defined above.

7. Intermediate compounds corresponding to the following formula: 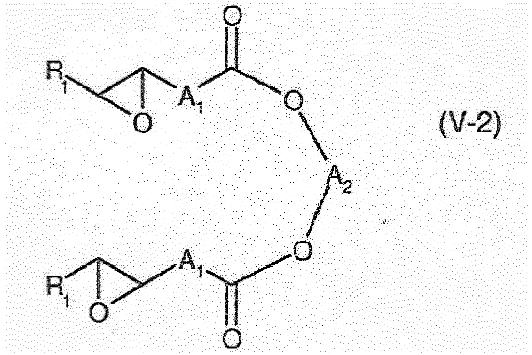 R₁, A₁ and A₂ being as defined in claim 1.

8. Polymers as obtained by polymerisation of a compound as defined in claim 6 and of a (poly)isocyanate.
